# EUROPEAN PATENT APPLICATION

(11) **EP 4 454 661 A1**
(43) Date of publication of application: **30.10.2024**
(21) Application number: 22911237.0
(22) Date of filing: 20.12.2022
(51) Int. Cl.: A61K 39/39, A61K 39/145, A61K 39/215, A61K 47/02, A61K 47/04, A61K 47/18, A61P 31/14, A61P 31/16

(54) **VACCINE ADJUVANT AGENT CONTAINING POLYACRYLIC ACID POLYMER AND USE OF SAME**

(30) Priority: 20.12.2021 JP 2021206279
(71) Applicant: Toko Yakuhin Kogyo Co., Ltd., Osaka-shi, Osaka 530-0022 (JP); Japan as Represented by Director-General of National Institute of Infectious Diseases, Tokyo 162-8640 (JP)
(72) Inventor: KAMISHITA, Taizou, Osaka-shi, Osaka 530-0022 (JP); MIYAZAKI, Takashi, Osaka-shi, Osaka 530-0022 (JP); SASAKI, Eita, Tokyo 162-8640 (JP)
(74) Representative: Hoffmann Eitle
(86) International application number: PCT/JP2022/046937
(87) International publication number: WO 2023/120535

(57) **Abstract**

The present disclosure provides a vaccine adjuvant agent comprising a polymer in which a constitutional unit is derived from acrylic acid, wherein the polymer has a carboxyl group content of 60.0 to 62.5 % by mass.

## Description

### TECHNICAL FIELD

The present invention relates to vaccine adjuvants and uses thereof.

### BACKGROUND ART

In vaccination, vaccine adjuvants are widely used to enhance the immunogenicity of vaccines when used in combination with antigens, and vaccine adjuvants such as aluminum salt adjuvants, oil emulsion adjuvants and the like are in practical use. On the other hand, some vaccine adjuvants have limited administration routes, and some have been abandoned for practical use due to toxicity or allergic reactions.

Although research and development of vaccine adjuvants have been actively conducted (Non-Patent Document 1, Patent Document 1), there is still a strong need to establish technology that enables a rapid development of vaccine antigens as well as a development of vaccine adjuvants to supplement them in response to an emergence of unknown viruses such as SARS-CoV-2 (COVID-19). In order to enable appropriate vaccine adjuvants to be selected depending on the administration route, formulation, etc., there is a strong demand in vaccine development for an increase in the options of vaccine adjuvants, particularly highly versatile vaccine adjuvants that can be used with a wide variety of antigens.

In pharmaceutical compositions, various polymers are used, as thickeners, adhesives, suspending agents, binders, emulsifiers, and the like. Known examples of polymers include acidic polymers such as sodium chondroitin sulfate, hyaluronic acid, and acrylic acid-based polymers; neutral polymers such as hypromellose and polyvinyl alcohol; and basic polymers such as chitin and chitosan.

### PRIOR ART DOCUMENT

### PATENT DOCUMENT

Patent Document 1: WO2009/004900

### NON-PATENT DOCUMENT

Non-patent Document 1: "Progress in Medicine (Igakunoayumi)", 2018, Vol. 264, No.5, pp.368-373
Non-patent Document 2: Vaccine, 1990, Vol. 8, No.6, pp.573-576

### SUMMARY OF THE INVENTION

### PROBLEMS TO BE SOLVED BY THE INVENTION

An objective of the present invention is to provide a highly versatile vaccine adjuvant agent that can be utilized with a wide variety of antigens.

### SOLUTIONS TO THE PROBLEMS

The present inventors, in view of the above problems, have conducted intensive research and have surprisingly found that, among many polymers, an acidic polymer having acrylic acid as a constituent unit with a carboxyl group content of 60.0 to 62.5 % by mass enhances the immunogenicity against a wide range of antigens, such as influenza virus, SARS-CoV-2, and ovalbumin, thereby achieving the present invention. In addition, the present inventors found that polymers within a certain range of molecular size (mass-average molecular weight in the case of non-crosslinked, polymer particle size in the case of crosslinked) have superior vaccine adjuvant effect, which led to the present invention. Specifically, the present disclosure provides the following aspects of the invention:
(Item 1)
   A vaccine adjuvant agent comprising a polymer in which a constitutional unit is derived from acrylic acid, wherein the polymer has a carboxyl group content of 60.0 to 62.5 % by mass.
(Item 2)
   The vaccine adjuvant agent according to Item 1, wherein the polymer is polyacrylic acid and/or a salt thereof.
(Item 3)
   The vaccine adjuvant agent according to Item 1 or 2, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is 0.05 to 10 µm.
(Item 4)
   The vaccine adjuvant agent according to any one of Items 1 to 3, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is adjusted to 0.05 to 10 µm by applying a mechanical shear force.
(Item 5)
   The vaccine adjuvant agent according to Item 4, wherein the carboxyl group content is 60.0 to 61.0 % by mass as calculated for the free form of the polyacrylic acid.
(Item 6)
   The vaccine adjuvant agent according to Item 5, wherein a number-based mode diameter of particles of the polymer is adjusted to 0.1 to 2.0 µm by applying a mechanical shear force.
(Item 7)
   The vaccine adjuvant agent according to Item 4, wherein the carboxyl group content is more than 61.0 % by mass and less than or equal to 62.0% by mass as calculated for the free form of the polyacrylic acid.
(Item 8)
   The vaccine adjuvant agent according to Item 7, wherein a number-based mode diameter of particles of the polymer is adjusted to 0.05 to 0.1 µm by applying a mechanical shear force.
(Item 9)
   The vaccine adjuvant agent according to Item 1 or 2, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is 0.1 to 50 µm.
(Item 10)
   The vaccine adjuvant agent according to Item 9, wherein the polymer has not been subjected to a mechanical shear force.
(Item 11)
   The vaccine adjuvant agent according to Item 1 or 2, wherein the polymer is a non-crosslinked polyacrylic acid and/or a salt thereof, and, the polymer has a mass-average molecular weight ranging from 50,000 to 300,000.
(Item 12)
   The vaccine adjuvant agent according to Item 11, wherein the carboxyl group content is 62.0 to 62.5 % by mass as calculated for the free form of the polyacrylic acid.
(Item 13)
   The vaccine adjuvant agent according to any one of Items 1 to 12, further comprising water.
(Item 14)
   The vaccine adjuvant agent according to any one of Items 3 to 9 and 13, wherein
   the vaccine adjuvant agent comprises the polymer and water, and optionally comprises at least one additive selected from the group consisting of sodium chloride, sodium hydroxide, and L-arginine, and
   the number-based mode diameter of particles of the polymer is adjusted by applying a mechanical shear force to a composition consisting of the polymer and the water, provided that, when the vaccine adjuvant agent comprises the at least one additive, the composition may comprise the at least one additive.
(Item 15)
   The vaccine adjuvant agent according to any one of Items 1 to 14, wherein sodium chloride is comprised in an amount of 0.1 to 1.5 % by mass based on the total mass of the vaccine adjuvant agent.
(Item 16)
   The vaccine adjuvant agent according to any one of Items 1 to 15, wherein the vaccine adjuvant agent further comprises sodium hydroxide and/or L-arginine
(Item 17)
   The vaccine adjuvant agent according to any one of Items 1 to 16, wherein the vaccine adjuvant agent further comprises L-arginine
(Item 18)
   The vaccine adjuvant agent according to any one of Items 1 to 17, wherein the vaccine adjuvant agent has pH of 6 to 8.
(Item 19)
   The vaccine adjuvant agent according to any one of Items 1 to 18, wherein the polymer is comprised in an amount of 0.01 to 50 % by mass based on the total mass of the vaccine adjuvant agent.
(Item 20)
   The vaccine adjuvant agent according to any one of Items 1 to 19, wherein the vaccine adjuvant agent further comprises at least one nonionic surfactant selected from the group consisting of an ether-type nonionic surfactant and an ester-ether type nonionic surfactant.
(Item 21)
   The vaccine composition according to Item 20, wherein the nonionic surfactant is polysorbate 80.
(Item 22)
   The vaccine adjuvant agent according to any one of Items 1 to 21, wherein the vaccine adjuvant agent further comprises at least one polyhydric alcohol selected from the group consisting of polyethylene glycol, glycerin, propylene glycol, and 1,3-butylene glycol.
(Item 23)
   The vaccine adjuvant agent according to Item 22, wherein the polyhydric alcohol is polyethylene glycol.
(Item 24)
   The vaccine adjuvant agent according to any one of Items 1 to 23, for enhancing an immune response to a pathogen-derived antigen.
(Item 25)
   The vaccine adjuvant agent according to Item 24, wherein a disease caused by the pathogen is a respiratory disease.
(Item 26)
   The vaccine adjuvant agent according to any one of Items 1 to 25, for enhancing an immune response to an antigen derived from an enveloped virus.
(Item 27)
   The vaccine adjuvant agent according to any one of Items 1 to 26, for enhancing an immune response to an antigen derived from at least one virus selected from the group consisting of coronavirus and influenza virus.
(Item 28)
   The vaccine adjuvant agent according to any one of Items 1 to 27, wherein the vaccine adjuvant agent is for administration via an injection route or a mucosal route.
(Item 29)
   The vaccine adjuvant agent according to any one of Items 1 to 28, wherein the dosage of the polymer per administration is 1 µg to 100 mg.
(Item 30)
   A method for enhancing an immune response to an antigen, comprising administering to a subject in need thereof the vaccine adjuvant agent according to any one of Items 1 to 29 in an amount effective as a vaccine adjuvant.
(Item 31)
   Use of the vaccine adjuvant agent according to any one of Items 1 to 29 for enhancing an immune response to an antigen.
(Item 32)
   A method for inducing an immune response to an antigen, comprising administering to a subject in need thereof the vaccine adjuvant agent according to any one of Items 1 to 29 in an amount effective as a vaccine adjuvant in combination with the antigen.
(Item 33)
   Use of the vaccine adjuvant agent according to any one of Items 1 to 29 for inducing an immune response to an antigen.
(Item 34)
   A method for preventing a disease caused by a pathogen, comprising administering to a subject in need thereof the vaccine adjuvant agent according to any one of Items 1 to 29 in an amount effective as a vaccine adjuvant in combination with an antigen derived from the pathogen.
(Item 35)
   Use of the vaccine adjuvant agent according to any one of Items 1 to 29 for preventing a disease caused by a pathogen.
(Item 36)
   Use of the vaccine adjuvant agent according to any one of Items 1 to 29 in the manufacture of a medicament for enhancing an immune response to an antigen, inducing an immune response to an antigen, or preventing a disease caused by a pathogen.
(Item 37)
   A vaccine composition comprising (i) the vaccine adjuvant agent according to any one of Items 1 to 29, and (ii) an antigen.
(Item 38)
   A vaccine composition comprising:
   (i) a polymer in an amount effective as a vaccine adjuvant, wherein a constitutional unit of the polymer is derived from acrylic acid, and the polymer has a carboxyl group content of 60.0 to 62.5 % by mass, and
   (ii) an antigen.
(Item 39)
   The vaccine composition according to Item 37 or Item 38, wherein the antigen is a pathogen-derived antigen.
(Item 40)
   The vaccine composition according to Item 39, wherein a disease caused by the pathogen is a respiratory disease
(Item 41)
   The vaccine composition according to any one of Items 37 to 40, wherein the antigen is an antigen derived from an enveloped virus (e.g., coronavirus and influenza virus).
(Item 42)
   The vaccine composition according to any one of Items 37 to 41, wherein the vaccine composition is for administration via an injection route or a mucosal route.
(Item 43)
   The vaccine composition according to any one of Items 37 to 42, wherein the polymer is comprised in a mass ratio of the antigen: the polymer = 1:0.1 to 1:1000.
(Item 44)
   A method for inducing an immune response to the antigen, comprising administering to a subject in need thereof an effective amount of the vaccine composition according to any one of Items 37 to 43.
(Item 45)
   Use of the vaccine composition according to any one of Items 37 to 43 for inducing an immune response to the antigen.
(Item 46)
   A method for preventing a disease caused by a pathogen, comprising administering to a subject in need thereof an effective amount of the vaccine composition according to any one of Items 37 to 43.
(Item 47)
   Use of the vaccine composition according to any one of Items 37 to 43 for preventing a disease caused by a pathogen.
(Item 48)
   The vaccine adjuvant agent according to any one of Items 1 to 25, or the vaccine composition according to any one of Items 37 to 43, further comprising polyethylene glycol (e.g., macrogol 400 and/or macrogol 4000).
(Item 49)
   The vaccine adjuvant agent according to any one of Items 1 to 25, or the vaccine composition according to any one of Items 37 to 43, further comprising a polyhydric alcohol such as glycerin, propylene glycol, 1,3-butylene glycol, and the like.
(Item 50)
   The vaccine adjuvant agent according to any one of Items 1 to 25, or the vaccine composition according to any one of Items 37 to 43 and 48, further comprising an ether-type, ester-ether type nonionic surfactant.
(Item 51)
   The vaccine composition according to Item 50, wherein the ether-type, ester-ether type nonionic surfactant is polysorbate 80 (polyoxyethylene sorbitan monooleate).

Any two or more of the elements described in the above [Item 1] to [Item 51] may be optionally selected and combined.

### EFFECTS OF THE INVENTION

According to the present invention, an immune response to an antigen may be enhanced, and an effective immune response may be induced with a smaller amount of antigen.

### DETAILED DESCRIPTION

In the present disclosure, when a numerical range is indicated as X to Y, it means "X or more and Y or less."

In one aspect, the present invention provides a homopolymer useful as a vaccine adjuvant wherein the constitutional unit is derived from acrylic acid, and the carboxyl group content is 60.0 to 62.5 % by mass (hereinafter, sometimes referred to as "the present invention polymer").

In this disclosure, a vaccine adjuvant refers to a substance that may enhance an immune response to an antigen when used in combination with the antigen.

In a certain embodiment, the present invention polymer is polyacrylic acid, a homopolymer consisting solely of main constitutional units derived from acrylic acid. The polyacrylic acid may be in the form of a salt, for example, a salt with sodium, potassium, ammonium ion, L-arginine, etc., and all or a part of the carboxyl groups derived from the acrylic acid may form a salt. Unless otherwise specified in this disclosure, the term "polyacrylic acid" may include salt forms.

In the present application, the term "carboxyl group content" means a ratio (% by mass) of carboxyl groups contained in a polymer relative to the total mass of the polymer.

When the present invention polymer is in the form of a salt, the carboxyl group content means a carboxyl group content determined by regarding the present invention polymer as a free form. It may be described as "the carboxyl group content is X % by mass as calculated for the free form of the polyacrylic acid", and this also means the carboxyl group content determined by regarding the polyacrylic acid in a salt form as a free form.

In the present application, a method for quantifying a carboxyl group content is not particularly limited, but for example, the method for quantifying carboxy vinyl polymer listed in the Japanese Pharmaceutical Excipients 2018 may be used.

The present invention polymer may be a crosslinked polymer, having a higher molecular weight by a crosslinking agent, or a non-crosslinked polymer, having no crosslinked structure.

In the present disclosure, when the cross-linked and non-cross-linked are not specified, the polymer may be both crosslinked and non-crosslinked types, unless the context indicates the contrary.

In a certain embodiment, the present invention polymer is a non-crosslinked polyacrylic acid, which has no crosslinked structures.

A linear, non-crosslinked polyacrylic acid has 62.5% by mass of the carboxyl group content, which is the maximum value of carboxyl group content. As the branched structure portion increases, the carboxyl group content decreases.

In a certain embodiment, the carboxyl group content of the non-crosslinked polyacrylic acid is 62.0 to 62.5 % by mass.

In a certain embodiment, the carboxyl group content of the non-crosslinked polyacrylic acid is 62.5 % by mass.

In a certain embodiment, the present invention polymer is a crosslinked polyacrylic acid. Examples of cross-linking agents for forming a cross-linked polymer include polyalkenyl ethers such as allyl pentaerythritol, allyl sucrose, allyl propylene, and the like and divinyl compounds of divinyl glycol, and the like, but are not limited to these.

In a certain embodiment, a cross-linking agent for forming a cross-linked polymer is at least one selected from the group consisting of allyl pentaerythritol, and allyl sucrose.

In a certain embodiment, a crosslinking agent for forming a crosslinked polymer is allyl pentaerythritol.

In a crosslinked polyacrylic acid, the carboxyl group content decreases as a three-dimensional structure is formed by, for example, a crosslinking agent.

In a certain embodiment, the carboxyl group content of the crosslinked polyacrylic acid is 60.0 to 62.0 % by mass.

In a certain embodiment, the present invention polymer is a crosslinked polyacrylic acid wherein the carboxyl group content is 60.0 to 61.0 % by mass.

In the present disclosure, a crosslinked polyacrylic acid having the carboxyl group content of 60.0 to 61.0 % by mass may be referred to as a highly crosslinked polyacrylic acid.

In a certain embodiment, the present invention polymer is a crosslinked polyacrylic acid wherein the carboxyl group content is more than 61.0 % by mass and less than or equal to 62.0 % by mass.

In the present disclosure, a crosslinked polyacrylic acid having the carboxyl group content of more than 61.0 % by mass and less than or equal to 62.0 % by mass may be referred to as a low crosslinked polyacrylic acid.

In a certain embodiment, the present invention polymer is a polyacrylic acid having a predetermined molecular size.

In a certain embodiment, the molecular size of non-crosslinked polyacrylic acid is defined by a mass-average molecular weight (Mw). The mass-average molecular weight of non-crosslinked polymer can be measured by a commonly used method. It can be determined, for example, by suitable physical measurements of a very dilute solution of the polymer. Commonly-used methods include gel permeation chromatography (GPC) and intrinsic viscosity. Light scattering, ultracentrifugation, and osmometry may also be used. If there is a difference in the values of the mass-average molecular weight depending on the measurement method, it is preferable to adopt the value measured by gel permeation chromatography (GPC).

In a certain embodiment, when the present invention polymer is a non-crosslinked polyacrylic acid and/or a salt thereof, the mass-average molecular weight is preferably 50,000 to 300,000, and more preferably 100,000 to 200,000, from the viewpoint of a more excellent immunity induction improving ability.

In a certain embodiment, when the present invention polymer is a non-crosslinked polyacrylic acid and/or a salt thereof, the carboxyl group content is 62.5% by mass and the mass-average molecular weight is 100,000 to 200,000, from the viewpoint of a more excellent immunity induction improving ability.

The above methods for measuring mass-average molecular weight generally require solubility of the polymer and therefore cannot generally be used to determine a molecular weight of insoluble crosslinked polyacrylic acid.

In a certain embodiment, the molecular size of crosslinked polyacrylic acid is defined by a particle size of the polymer. In a certain embodiment, the measurement result of the polymer particle size is specified as a number-based mode diameter determined by a laser diffraction particle size distribution analyzer. Unless otherwise specified, the "mode diameter" in this disclosure means the " a number-based mode diameter determined by a laser diffraction particle size distribution analyzer."

In a certain embodiment, a polymer particle size of (particle size distribution) is determined using a number-based particle size distribution, which can be measured using a laser diffraction particle size distribution analyzer, and it is preferable that the result is not significantly different from the volume-based particle size distribution result. If at least one device shows that a mode diameter of particles of a polymer falls within a predetermined range, it may be regarded that the mode diameter of particles of the polymer is within the predetermined range.

In addition, if it is confirmed that the measurement value obtained by a dynamic light scattering particle size distribution analyzer is equivalent to the number-based mode diameter determined by a laser diffraction particle size distribution analyzer, then the measurement value of the dynamic light scattering particle size distribution analyzer may be considered as the number-based mode diameter determined by a laser diffraction particle size distribution analyzer. Furthermore, if it is confirmed that the measurement value obtained by observing the state of the polymer particles using a phase contrast microscope, Opt-SEM (Optical Shadow Effect Mode Microscope), or similar instruments is equivalent to the number-based mode diameter determined by a laser diffraction particle size distribution analyzer, then a mode diameter may be determined by such method.

Additionally, a polymer particle size may be measured using a laser diffraction particle size distribution analyzer (for example, Shimazu SALD-2300, Shimazu SALD-7000, or similar instruments) according to the method described in Test Examples in the present application.

A particle size (mode diameter) of particles of a polymer may be adjusted by applying an external mechanical shear force to the present invention polymer (or an agent/composition containing the present invention polymer). For example, the mode diameter of polymer particles may be appropriately adjusted by applying an external shear force (a mechanical shear force) to a commercially available acrylic acid-based polymer. The operation of applying the shear force can be carried out by a method known to those skilled in the art. Examples of devices that can be used to apply a mechanical shear force include a high-speed spinning-type emulsifying device, a colloidal mill-type emulsifying device, a high-pressure emulsifying device, a roll mill-type emulsifying device, an ultrasonic-type emulsifying device and a membrane-type emulsifying device. Especially, a homo mixer-type, a comb-type, and an intermittently-jet-stream-generating-type high-speed spinning-type emulsifying device are preferable. An intermittently jet-stream-generating-type high-speed spinning-type emulsifying device is particularly preferred.

In a certain embodiment, "a number-based mode diameter of particles of the polymer is adjusted to a predetermined range (e.g., 0.05 to 10 µm) by applying a mechanical shear force" refers to that the number-based mode diameter of particles of the polymer is adjusted to be within a predetermined range by applying a mechanical shear force to an agent/composition containing the polymer.

A particle size of particles of a polymer is preferably measured by appropriately mixing the polymer with other ingredients (neutralizing agent, water, etc.) which may be contained in the present invention adjuvant agent described below, however it is preferable that substances that may significantly affect the measured particle size (e.g., surfactants, polyhydric alcohols, insoluble/slightly soluble substances) are not mixed. It is preferable that the mixture for measuring the particle size is in a uniform solution state after mixing.

For example, it may be measured in the following manner.

When variations in particle size measurements may occur due to the concentration of the polymer, it is desirable that the polymer concentration in the sample used for measuring particle size is close to the polymer concentration in the present invention adjuvant agent. When the present invention adjuvant agent contains water, it is preferable to determine the particle size (mode diameter) of the polymer particles by measuring a mixture containing at least the water (for example., the total amount of the water). When the present invention adjuvant agent contains a pH adjuster (such as sodium hydroxide, L-arginine), it is preferable to measure the particle size (mode diameter) of the polymer particles by measuring the mixture containing the pH adjuster (for example, to reach a predetermined pH). When the present invention adjuvant agent contains sodium chloride, it is preferable to measure the particle size (mode diameter) of the polymer particles by measuring the mixture containing sodium chloride (for example, the total amount of the sodium chloride).

In a certain embodiment, a mode diameter of particles of a crosslinked polyacrylic acid is 0.05 to 50 µm.

In a certain embodiment, the present invention polymer is a crosslinked polyacrylic acid that has not been subjected to a mechanical shear force.

In one embodiment, the present invention polymer is a crosslinked polyacrylic acid that has not been subjected to a mechanical shear force and has a mode diameter of polymer particles of 0.1 to 50 µm.

In a certain embodiment, the present invention polymer is a crosslinked polyacrylic acid that has not been subjected to a mechanical shear force, and a mode diameter of particles of the polymer of 1 to 15 µm.

In a certain embodiment, the present invention polymer is a crosslinked polyacrylic acid that has been subjected to a mechanical shear force.

In a certain embodiment, from the viewpoint of a more excellent immunity induction improving ability, the present invention polymer is a crosslinked polyacrylic acid that has been subjected to a mechanical shear force, a mode diameter of particles of the polymer is 0.05 to 10 µm (e.g., 0.1 to 2.0 µm, 0.2 to 2.0 µm, 0.2 to 1.0 µm, 0.5 to 1.0 µm, 0.7 to 1.0 µm).

In a certain embodiment, the present invention polymer is a crosslinked polymer, wherein the crosslinked polymer is treated in an agent/composition containing other ingredient(s) (e.g., water, etc.) by applying an external shear force (a mechanical shear force) so that the mode diameter of particles of the polymer in the agent/composition falls within the range of 0.05 to 10 µm (e.g., 0.1 to 2 µm, 0.2 to 2.0 µm, 0.2 to 1.0 µm, 0.5 to 1.0 µm, 0.7 to 1.0 µm).

In a certain embodiment, the present invention polymer is a highly crosslinked polyacrylic acid having the carboxyl group content of 60.0 to 61.0 % by mass, wherein the highly crosslinked polyacrylic acid is treated in an agent/composition containing other ingredient(s) (preferably water or an aqueous NaCl solution) by applying an external shear force (a mechanical shear force) so that the mode diameter of particles of the polymer in the agent/composition falls within the range of 0.05 to 10 µm (e.g., 0.1 to 2.0 µm, 0.2 to 2.0 µm, 0.2 to 1.0 µm, 0.5 to 1.0 µm, 0.7 to 1.0 µm).

In a certain embodiment, the present invention polymer is a low crosslinked polyacrylic acid having the carboxyl group content of more than 61.0 % by mass and less than or equal to 62.0 % by mass, wherein the low crosslinked polyacrylic acid is treated in an agent/composition containing other ingredient(s) (e.g., water, etc.) by applying an external shear force (a mechanical shear force) so that the mode diameter of particles of the polymer in the agent/composition falls within the range of 0.05 to 10 µm (e.g., 0.05 to 1 µm, 0.05 to 0.1 µm).

The dosage of the present invention polymer is not particularly limited as long as it is an amount that enhances the immune response to an antigen, and it may vary depending on the antigen, the dosage form of the composition containing the present invention polymer, the administration route, the subject of administration, and the like. For example, the dosage of the present invention polymer per administration is from 1 µg to 1000 mg, preferably from 10 µg to 100 mg per administration, and more preferably from 100 µg to 10 mg per administration. Furthermore, for example, the dosage of the present invention polymer is in the range of an antigen-to-the present invention polymer mass ratio of 1:0.1 to 1:1000, more preferably 1:10 to 1:500.

In a certain embodiment, the dosage of the polymer is from 1 µg to 100 mg per administration.

In a certain embodiment, the present invention polymer is administered by injection (e.g., intradermally, subcutaneously, intraperitoneally) with the dosage per administration being 1 µg to 10 mg, preferably 1 µg to 1 mg.

In a certain embodiment, the present invention polymer is administered mucosally (e.g., intranasally) and the dosage per administration is 10 µg to 100 mg, preferably between 100 µg to 10 mg.

In a certain aspect, the present invention provides a vaccine adjuvant agent comprising the present invention polymer (hereinafter, may be referred to as " the present invention adjuvant agent").

The above description regarding the present invention polymer may be used as a description regarding the present invention polymer contained in the present invention adjuvant agent.

The present invention adjuvant agent may be the present invention polymer itself, or may be a composition further containing other ingredient(s) such as water.

In a certain embodiment, the present invention adjuvant agent is used as an additive in a pharmaceutical composition containing other ingredient(s) (e.g., an antigen, etc.).

In a certain embodiment, the present invention adjuvant agent is a vaccine adjuvant formulation that does not contain an antigen.

The administration route of a vaccine adjuvant formulation is not particularly limited as long as it may exert the effect of enhancing an immune response to an antigen. For example, oral administration and parenteral administration (such as injection or transmucosal administration) are included. The present invention adjuvant agent may, for example, be administered separately, without being mixed with the antigen. The administration route of the present invention adjuvant agent may be the same as or different from that of an antigen. The present invention adjuvant may, for example, be mixed with an antigen at the time of use and administered. Preferably, the present invention adjuvant agent is mixed with an antigen and administered. The present invention adjuvant agent may usually be administered to a living body simultaneously with an antigen, but may be administered before or after administration of the antigen. In the cases where the present invention adjuvant agent is administered simultaneously with an antigen, it may be administered substantially simultaneously with the antigen. For example, the present invention adjuvant agent and an antigen may be administered to a subject completely simultaneously, or may be administered consecutively within a certain period of time (preferably within a few minutes).

The amount of the present invention polymer contained in the present invention adjuvant agent may vary depending on the formulation, method of use, administration route, antigen used in combination, and the subject to be administered. For example, it ranges from 0.01 to 100 % by mass relative to the total mass of the present invention adjuvant agent, preferably from 0.01 to 50 % by mass, more preferably 0.01 to 10 % by mass, even more preferably, 0.1 to 5 % by mass.

The present invention adjuvant agent may contain one or more types of the present invention polymer. When the above-mentioned present invention adjuvant agent contains two or more types of the present invention polymer, the amount of the present invention polymer contained in the present invention adjuvant agent refers to the total amount of the two or more types of the present invention polymer.

The present invention adjuvant agent may be produced by mixing the present invention polymer and other ingredients as appropriate, using a method commonly used for producing pharmaceutical compositions or additives for pharmaceutical compositions. The mode diameter of particles of the polymer may be appropriately adjusted to within a preferred range by applying an external shear force (a mechanical shear force) during the production process of the present invention adjuvant agent using the present invention polymer having a large mode diameter.

In a certain embodiment of the present invention adjuvant agent, the mode diameter of particles of the polymer is measured at a polymer concentration equivalent to the concentration when mixed with the total amount of water that may be contained in the present invention adjuvant agent.

In a certain embodiment of the present invention adjuvant agent, the mode diameter of particles of the polymer is measured under conditions in which the polymer concentration is equivalent to that when mixed with the total amount of water contained in the present invention adjuvant agent and the pH is adjusted to that of the present invention adjuvant agent (with a pH adjuster as necessary).

The pH of the present invention adjuvant agent is not particularly limited and may be appropriately adjusted depending on, for example, the formulation, method of use, administration route, antigen used in combination, and the like. The pH may be adjusted using an appropriate acidic substance (phosphoric acid, citric acid, hydrochloric acid, etc.) or an appropriate basic substance (sodium hydroxide, potassium hydroxide, a basic amino acid such as lysine and arginine, etc.). Various buffer solutions adjusted to be acidic or alkaline may also be used.

In a certain embodiment, the pH of the present invention adjuvant agent is 6 to 8 (preferably 6.5 to 7.5).

In a certain embodiment, the present invention adjuvant agent comprises water.

In a certain embodiment, the present invention adjuvant agent comprises at least 50 % by mass (for example, at least 70 % by mass, at least 80 % by mass, at least 90 % by mass) of water relative to the total mass of the present invention adjuvant agent.

In a certain embodiment, the present invention adjuvant agent comprises sodium hydroxide and/or L-arginine. In a certain embodiment, the present invention adjuvant agent is adjusted to a pH6 to 8 (preferably 6.5 to 7.5) with sodium hydroxide and/or L-arginine. In a certain embodiment, the present invention adjuvant is neutralized with sodium hydroxide and/or L-arginine. The order of adjusting the pH and applying a mechanical shear force is not particularly limited. In a certain embodiment, the pH is adjusted to pH6 to 8 (preferably 6.5 to 7.5) prior to application of mechanical shear force. In a certain embodiment, a mechanical shear force is applied before the pH is adjusted to pH 6-8 (preferably 6.5-7.5).

In a certain embodiment, the present invention adjuvant agent comprises sodium chloride.

In a certain embodiment, the present invention adjuvant agent comprises 0.1 to 1.5 % by mass (for example, 0.2 to 1.0 % by mass) of sodium chloride relative to the total mass of the present invention adjuvant agent.

In a certain embodiment, the present invention adjuvant agent comprises a polymer which is a crosslinked polyacrylic acid and/or a salt thereof wherein the crosslinked polyacrylic acid and/or a salt thereof has a carboxyl group content of 60.0 to 62.0 % by mass (preferably, 60.0 to 61.0 % by mass) as calculated for the free form of the polyacrylic acid; and water,
and, optionally comprises at least one additive selected from the group consisting of sodium chloride, sodium hydroxide, and L-arginine;
wherein a number-based mode diameter of particles of the polymer is 0.05 to 50 µm (preferably, 0.05 to 10 µm, 0.1 to 2 µm, 0.2 to 2.0 µm, 0.2 to 1.0 µm, 0.5 to 1.0 µm, 0.7 to 1.0 µm), wherein the number-based mode diameter of particles of the polymer is adjusted by applying a mechanical shear force to a composition consists of the polymer and the water, provided that, when the vaccine adjuvant agent comprises the at least one additive, the composition may comprise the at least one additive. Here, when the carboxyl group content is 60.0 to 61.0 % by mass as calculated for the free form of the polyacrylic acid, the number-based mode diameter of particles of the polymer is preferably adjusted to 0.05 to 10 µm (for example, 0.1 to 2.0 µm, 0.2 to 2.0 µm, 0.2 to 1.0 µm, 0.5 to 1.0 µm, 0.7 to 1.0 µm). Here, when the carboxyl group content is more than 61.0 % by mass and less than or equal to 62.0 % by mass as calculated for the free form of the polyacrylic acid, the number-based mode diameter of particles of the polymer is preferably adjusted to 0.05 to 10 µm (for example, 0.05 to 1 µm, 0.05 to 0.1 µm). Here, the "number-based mode diameter of particles of the polymer" means a number-based mode diameter of particles of the polymer when the composition is measured as a sample. Here, sodium hydroxide and L-arginine may be added as a pH adjuster to adjust the pH of the composition to 6 to 8 (preferably 6.5 to 7.5). Here, the composition comprises the entire amount of the polymer. Here, it is preferable that the entire amount of water is contained in the composition. However, when the water is used as a solvent for other ingredients such as a surfactant, the water contained in the composition does not have to be the entire amount of water in the present invention adjuvant agent. Here, the present invention adjuvant agent contains at least 50 % by mass (for example, at least 70 % by mass, at least 80 % by mass, at least 90 % by mass) of water relative to the total mass of the present invention adjuvant agent.

Here, the present invention adjuvant agent may contain at least one additive selected from the group consisting of a nonionic surfactant and a polyhydric alcohol.

In a certain embodiment, the present invention adjuvant agent consists of the present invention polymer and water, and optionally comprises at least one additive selected from the group consisting of sodium chloride, sodium hydroxide, and L-arginine, and optionally comprises at least one additive selected from the group consisting of a nonionic surfactant and a polyhydric alcohol.

The viscosity of the present invention adjuvant agent is not particularly limited and may be appropriately adjusted depending on, for example, the formulation, method of use, administration route, and the like. The method for adjusting the viscosity is not particularly limited, and may be a commonly used method such as adding a viscosity modifier or applying an external shear. The viscosity of the present invention adjuvant agent is, for example, 5 to 5000 mPa·s, preferably 5 to 1000 mPa·s, more preferably 5 to 500 mPa·s.

In a certain aspect, the present invention provides a vaccine composition comprising (i) the present invention adjuvant agent, and (ii) an antigen. (hereinafter, sometimes referred to as " the present invention vaccine composition")

In a certain aspect, the present invention provides a vaccine composition comprising (i) the present invention polymer, and (ii) an antigen. (hereinafter, sometimes referred to as " the present invention vaccine composition")

The amount of the present invention polymer contained in the present invention vaccine composition is not particularly limited, so long as it is an effective amount as a vaccine adjuvant, and may be appropriately selected depending on the formulation, administration route, antigen, subject to be administered, etc.

In this disclosure, the term "in an amount effective as a vaccine adjuvant" refers to an amount that enhances an immune response to an antigen. For example, it means the amount of the present invention polymer that shows an improvement in the immune response to an antigen compared to a vaccine composition that does not contain the present invention polymer.

The amount of the present invention polymer contained in the present invention vaccine composition may be, for example, 0.001 to 10 % by mass, preferably 0.01 to 2.5 % by mass, based on the total mass of the present invention vaccine composition. The amount of the present invention polymer contained in the present invention vaccine composition may be 0.05 to 5 % by mass, more preferably 0.1% to 2.5 % by mass, based on the total mass of the present invention vaccine composition.

The amount of the present invention polymer contained in the present invention vaccine composition is, for example, in a mass ratio of antigen: the present invention polymer =1:0.1 to 1:1000, more preferably 1:10 to 1:500.

The amount of an antigen contained in the present invention vaccine composition is not particularly limited, and may be appropriately selected depending on the formulation, administration route, method of use, type of antigen, subject to be administered, and the like. For example, it is 0.0001 to 10.0 % by mass, 0.01 to 10.0 % by mass, more preferably 0.05 to 5.0 % by mass, based on the total mass of the present invention vaccine composition.

In the present invention, the amount of an antigen to be administered is not particularly limited as long as it is an amount sufficient to induce an antigen-specific antibody [IgA, IgG, etc.] when used in combination with the present invention polymer, and may vary depending on the type of antigen, target disease, administration route, subject to be administered, etc. For example, the dose of antigen per administration is between 0.1 µg to 10 mg, preferably1 µg to 5 mg, more preferably 1 µg to 1 mg per administration.

The present invention vaccine composition may contain one or more types of the present invention polymer. When the present invention vaccine composition contains two or more types of the present invention polymer, the amount of the present invention polymer in the present invention vaccine composition refers to the total amount of the two or more types of the present invention polymer.

The present invention vaccine composition may contain one or more types of antigens. When the present invention vaccine composition contains two or more types of antigens, the amount of antigen contained in the present invention vaccine composition refers to the total amount of the two or more types of antigens.

The method for producing the present invention vaccine composition is not particularly limited. The present invention vaccine composition may be produced by a method commonly used for a production of vaccine composition. For example, the present invention vaccine composition may be prepared by dissolving or suspending an antigen in a physiological saline, an appropriate buffer such as phosphate-buffered saline, or the like, and then gently mixing the resulting mixture with the present invention polymer/the present invention adjuvant agent until uniform.

The pH of the present invention vaccine composition is not particularly limited and may be appropriately adjusted depending on the formulation, method of use, administration route, antigen, etc. The pH can be adjusted using an appropriate acidic substance (phosphoric acid, citric acid, hydrochloric acid, etc.) or a basic substance (sodium hydroxide, potassium hydroxide, a basic amino acid such as lysine and arginine, etc.). Various buffer solutions adjusted to be acidic or alkaline may also be used.

In a certain embodiment, the pH of the present invention vaccine composition is 6 to 8 (preferably 6.5 to 7.5).

In a certain embodiment, the present invention vaccine composition comprises sodium hydroxide and/or L-arginine. In a certain embodiment, the present invention vaccine composition is neutralized with sodium hydroxide and/or L-arginine.

In a certain embodiment, the present invention vaccine composition comprises sodium chloride.

In a certain embodiment, the present invention vaccine composition comprises 0.05 to 1.5 % by mass (for example, 0.1 to 1.0 % by mass) of sodium chloride relative to the total mass of the present invention vaccine composition.

The viscosity of the present invention vaccine composition is not particularly limited and may be appropriately adjusted depending on the formulation, method of use, administration route, and the like. The method for adjusting the viscosity is not particularly limited, and may be a commonly used method such as adding a viscosity modifier or applying external shear. The viscosity of the present invention vaccine composition is, for example, 5 to 5000 mPa·s, preferably 5 to 1000 mPa·s, more preferably 5 to 500 mPa·s.

In the present invention, the viscosity can be measured by a commonly used method. A preferred method is to use a rotational viscometer.

The administration route of the present invention vaccine composition is not particularly limited, and may be oral or parenteral (e.g., injection or mucosal administration). Examples of mucous membranes to which the present invention vaccine composition may be applied include mucous membranes of the nasal cavity, oral cavity, pharynx, trachea, lungs, and intestinal tract. The present invention vaccine composition may be administered, for example, by injection intradermally subcutaneously, intramuscularly, or intraperitoneally; by application, instillation, or spray into the oral cavity; by instillation or spray into the nasal cavity; or by aerosol or dry powder inhalation into the lungs. The present invention vaccine composition may, for example, be a nasal administration formulation, with target sites including the nasal mucosa and nasopharynx.

The present invention vaccine composition may be a formulation that is administered without dilution or a formulation that is appropriately diluted at the time of use.

The present invention adjuvant agent/the present invention vaccine composition may contain an active ingredient, diluent, bactericide, preservative, surfactant, stabilizing agent and the like as long as they can be used in combination.

The present invention adjuvant agent/the present invention vaccine composition may contain polyethylene glycol (e.g., macrogol 400, macrogol 4000, macrogol 20000) (e.g., 0.05 to 15 % by mass, 0.1 to 10 % by mass, 0.1 to 5 % by mass, relative to the total mass of the present invention adjuvant agent/the present invention vaccine composition).

In a certain embodiment, the present invention adjuvant agent/the present invention vaccine composition may contain at least one additive selected from the group consisting of a nonionic surfactant and a polyhydric alcohol, from the viewpoint of a more excellent ability to improve immune induction.

In the present invention adjuvant agent, when the number-based mode diameter of particles of the polymer is adjusted by applying a mechanical shear force, it is preferable to add the additives after the mechanical shear force is applied, as the additives may affect the mode diameter.

Examples of nonionic surfactants include ether-type nonionic surfactants and ester-ether type nonionic surfactants, and more specific examples include polysorbates and polyoxyethylene hydrogenated castor oils, and a preferred example is polysorbate 80 (also known as polyoxyethylene sorbitan monooleate).

Examples of the content of nonionic surfactant(s) include 0.01 to 1 % by mass, 0.1 to 0.7 % by mass, based on the total mass of the present invention adjuvant agent/the present invention vaccine composition.

Examples of polyhydric alcohols include polyethylene glycol (for example, macrogol 400, macrogol 4000, macrogol 20000), glycerin, propylene glycol, and 1,3-butylene glycol.

Examples of the content of polyhydric alcohol(s) include 0.1 to 10 % by mass, 0.1 to 5 % by mass, based on the total mass of the present invention adjuvant agent/the present invention vaccine composition.

In a certain embodiment, the present invention adjuvant agent comprises a polymer which is a crosslinked polyacrylic acid and/or a salt thereof, wherein the crosslinked polyacrylic acid and/or a salt thereof has a carboxyl group content of 60.0 to 61.0 % by mass as calculated for the free form of the polyacrylic acid, and wherein a number-based mode diameter of particles of the polymer is adjusted to 0.05 to 10 µm (for example, 0.1 to 2.0 µm, 0.2 to 2.0 µm, 0.2 to 1.0 µm, 0.5 to 1.0 µm, 0.7 to 1.0 µm) by applying a mechanical shear force. The present invention adjuvant agent may optionally comprise at least one additive selected from the group consisting of a nonionic surfactant and a polyhydric alcohol, but the at least one additive is added after adjusting the mode diameter.

In a certain embodiment, the present invention adjuvant agent comprises a polymer which is a crosslinked polyacrylic acid and/or a salt thereof, wherein the crosslinked polyacrylic acid and/or a salt thereof has a carboxyl group content of 60.0 to 61.0 % by mass as calculated for the free form of the polyacrylic acid; and water,
wherein a number-based mode diameter of particles of the polymer in the presence of the total amount of water is adjusted to 0.05 to 10 µm (for example, 0.1 to 2.0 µm, 0.2 to 2.0 µm, 0.2 to 1.0 µm, 0.5 to 1.0 µm, 0.7 to 1.0 µm) by applying a mechanical shear force. The present invention adjuvant agent may optionally comprise at least one additive selected from the group consisting of a nonionic surfactant and a polyhydric alcohol, but the at least one additive is added after adjusting the mode diameter.

In a certain embodiment, the present invention adjuvant agent comprises a polymer which is a crosslinked polyacrylic acid and/or a salt thereof, wherein the crosslinked polyacrylic acid and/or a salt thereof has a carboxyl group content of 60.0 to 61.0 % by mass as calculated for the free form of the polyacrylic acid; water; and a pH adjuster (for example, sodium hydroxide, L-arginine),
wherein the polymer and water are mixed, and the resulting mixture is adjusted to a pH of 6 to 8 (preferably 6.5 to 7.5) using the pH adjuster, and then the resulting mixture is subjected to a mechanical shear force, adjusting a number-based mode diameter of particles of the polymer to 0.05 to 10 µm (for example, 0.1 to 2.0 µm, 0.2 to 2.0 µm, 0.2 to 1.0 µm, 0.5 to 1.0 µm, 0.7 to 1.0 µm). The present invention adjuvant agent may optionally comprise at least one additive selected from the group consisting of a nonionic surfactant and a polyhydric alcohol, but the at least one additive is added after adjusting the mode diameter.

In a certain aspect, the invention provides a method for enhancing an immune response to an antigen, comprising administering to a subject in need thereof an effective amount of the present invention polymer/the present invention adjuvant agent.

In a certain aspect, the invention provides a use of the present invention polymer/the present invention adjuvant agent for enhancing an immune response to an antigen.

In a certain aspect, the present invention provides a method for inducing an immune response to an antigen, comprising administering to a subject in need thereof an effective amount of the present invention polymer/the present invention adjuvant agent in combination with an antigen.

In a certain aspect, the present invention provides a use of the present invention polymer/the present invention adjuvant agent for inducing an immune response to an antigen.

In a certain aspect, the present invention provides a method for preventing a disease caused by a pathogen, comprising administering to a subject in need thereof an effective amount of the present invention polymer/the present invention adjuvant agent in combination with an antigen derived from the pathogen.

In a certain aspect, the present invention provides a use of the present invention polymer/the present invention adjuvant agent in the prevention of a disease caused by a pathogen.

In a certain aspect, the present invention provides a use of the present invention polymer in the production of the present invention adjuvant agent/the present invention vaccine composition.

In a certain aspect, the present invention provides a use of the present invention adjuvant agent in the production of the present invention vaccine composition.

In the present disclosure, inducing an immune response to an antigen means that the production of at least one type of antigen-specific antibody (IgA, IgG, etc.) is induced.

In the present disclosure, enhancing an immune response to an antigen means increasing the production of at least one type of antigen-specific antibody (IgA, IgG, etc.), and may be evaluated, for example, by comparison with a vaccine composition not containing the present invention polymer.

Since antigen-specific IgG2a is closely related to helper T cell type 1 (Th1) cellular immunity, it is preferable that the present invention adjuvant agent is effective in enhancing the production of antigen-specific IgG2a.

Since antigen-specific IgG and IgA have different functions, it is preferable that the production of both is enhanced. In a certain embodiment, the present invention adjuvant agent may be used to enhance the production of both antigen-specific IgG and IgA.

Preventing a disease caused by a pathogen may include not only preventing infection/onset of the disease, but also suppressing the development of the disease, alleviating symptoms of the disease, and preventing the recurrence of the disease.

In this disclosure, an "effective amount" of the present invention vaccine composition may refer to the amount of the present invention vaccine composition required to provide a benefit to a subject in inducing an immune response to an antigen/preventing a disease caused by a pathogen.

In the present invention, the subject is not particularly limited, and examples thereof include humans and non-human animals, such as poultry (e.g., chickens, ducks, etc.), livestock (e.g., cattle, pigs, etc.), and pets (e.g., dogs, cats). Preferably, the subject is a human.

In the present invention, the pathogen refers to a bacterium, a virus, a mycoplasma, and the like that may cause a disease in a subject. Examples of the pathogens include coronavirus (e.g., pathogens of severe acute respiratory syndrome (SARS), e.g., SARS-CoV-2), influenza virus, hepatitis B virus, hepatitis C virus, human immunodeficiency virus (HIV), chickenpox virus, measles virus, mumps virus, poliovirus, rotavirus, adenovirus, herpes virus, human papillomavirus, rubella virus, Streptococcus pneumoniae, Mycobacterium tuberculosis, Bordetella pertussis, Neisseria meningitidis, Haemophilus influenzae type b, Vibrio cholerae, Corynebacterium diphtheriae, and mycoplasma.

In the present invention, the antigen is not particularly limited, and examples include antigens derived from a pathogen (pathogen-derived antigens) and tumor-associated antigens. Examples of pathogen-derived antigens include antigens derived from the pathogens listed above.

In certain embodiment of the invention, the pathogen-derived antigen is an antigen derived from an enveloped virus.

In a certain embodiment of the invention, the pathogen-derived antigen is an antigen derived from at least one pathogen selected from the group consisting of coronavirus and influenza virus.

In a certain embodiment of the invention, the pathogen-derived antigen is an antigen derived from SARS-CoV-2.

In the present invention, the pathogen-derived antigen may be any pathogen-derived antigen usually used in vaccine preparations.

Examples of the pathogen-derived antigen include natural products purified from pathogens, and proteins, glycoproteins, peptides, polysaccharides, lipopolysaccharides, polynucleotides, or DNAs encoding antigens that are artificially produced by techniques such as genetic recombination.

Examples of pathogen-derived antigens include complete virus particles (virions), incomplete virus particles, virion constituent particles, viral nonstructural proteins, proteins or glycoproteins derived from pathogens, infection-protective antigens, and epitopes for neutralization reactions, and the like. These may include both those with infectivity (live antigens) and those that have lost infectivity (inactivated antigens). Examples of pathogen-derived antigens also include component vaccines, subunit vaccines, vector vaccines, and genetic vaccines.

### Examples

The present invention will be further described in detail with reference to Examples, Comparative Examples, and Test Examples. However, the present invention is not limited to these examples.

In the Examples, "polyacrylic acid" refers to the free form of polyacrylic acid. "Mode diameter" is the result based on the number.

### [Test Example 1] Antibody Production Induction Test in Mice (Intranasal mucosal administration, Influenza split antigen, Part 1)

### 1.Preparation of vaccine formulation

### (1) Preparation of antigen stock solution

Influenza split antigen [H1N1] was mixed with physiological saline until homogeneous to obtain an antigen stock solution (antigen concentration: 0.0066HA w/v%).

### (2)-1 Preparation of polymer-containing compositions used in sample numbers A#03 to A#10

Each polymer was placed in purified water or an aqueous sodium chloride solution, neutralized with sodium hydroxide or L-arginine, and mixed until homogeneous to obtain a composition. For the polymer-containing compositions "with mechanical shear treatment", the obtained composition was subjected to a mechanical shear force using an intermittently-jet-stream-generating-type high-speed spinning-type emulsifying device to adjust the particle size of the polymer contained in the polymer-containing composition.

### (2)-2 Preparation of polymer-containing compositions used in sample numbers A#11 to A#19

Each polymer was mixed with purified water until homogeneous to obtain a composition.

For A#12, L-arginine was added (0.375% by mass based on the polymer-containing composition).

### (2)-3 Preparation of Publicly known vaccine adjuvant-containing compositions used in sample numbers A#20 to A#23

Each publicly known vaccine adjuvant (Poly I:C, CpG K3, R848, or aluminum hydroxide gel) was mixed with physiological saline until homogeneous to obtain a composition.

### (2)-4 Measurement of mode diameter of polymer particles

The mode diameter of each polymer before mixing with the antigen was measured using a laser diffraction particle size distribution analyzer (Shimazu SALD-2300, Shimazu SALD-7000).

For the polymer-containing composition subjected to mechanical shear force, the mode diameter of particles of the polymer was measured by a laser diffraction particle size distribution analyzer after applying the mechanical shear force to the polymer-containing composition.

The table below shows details of the polymer used in the preparation of the vaccine formulation and the measurement results of the mode diameter of particles of the polymer; as well as the neutralizing agent used in the preparation of the polymer-containing composition, the sodium chloride concentration relative to the total amount of the polymer-containing composition, and whether or not mechanical shear treatment was applied.

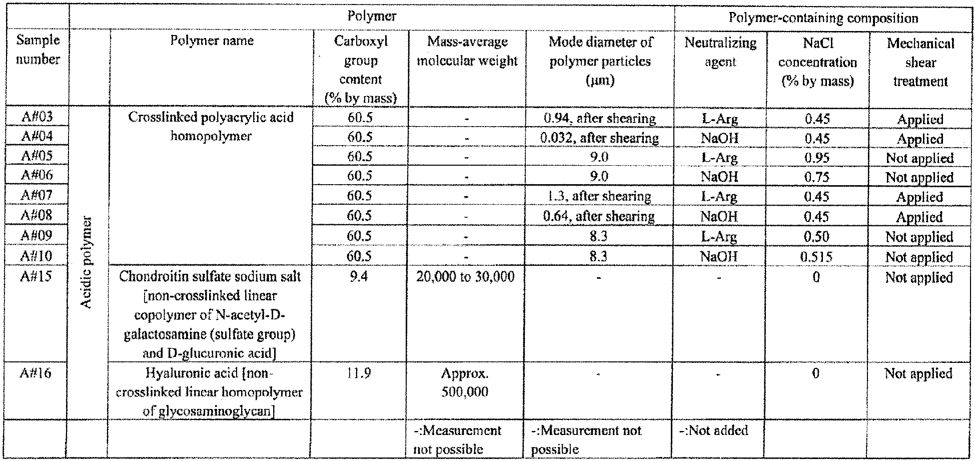

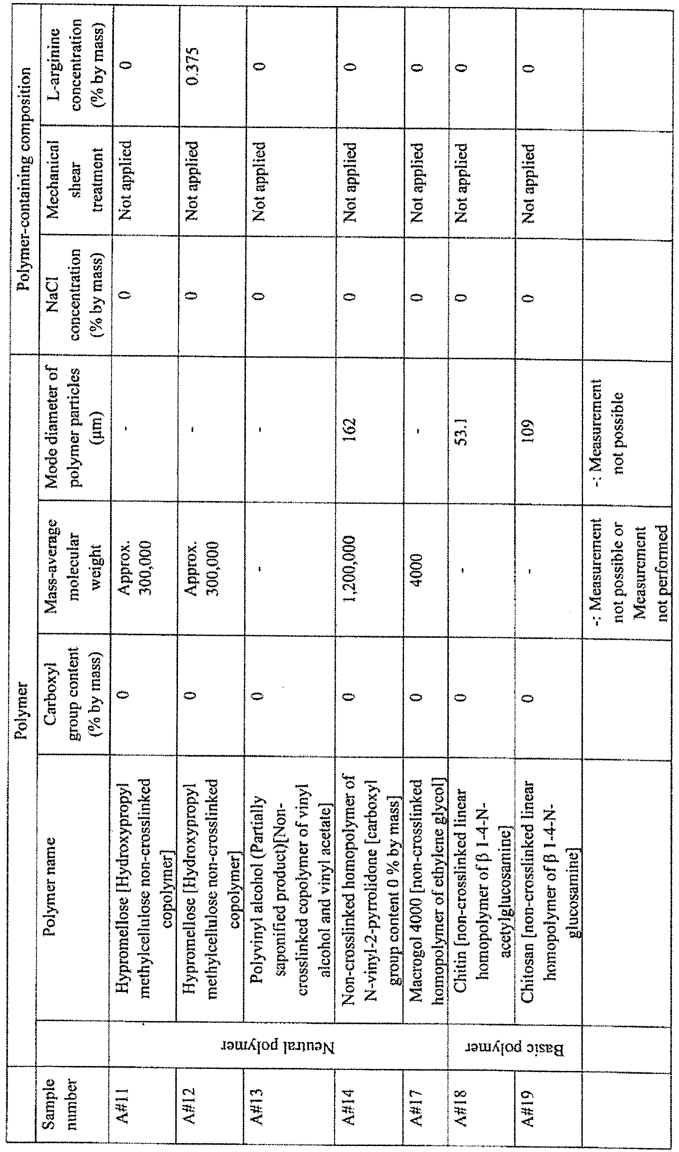

### (3)-1 Polymer-free vaccine formulation (Sample numbers A#01)

The antigen stock solution and physiological saline were mixed in a 1:1 volume ratio to obtain a polymer-free vaccine formulation with an antigen concentration of 0.0033HA w/v%.

### (3)-2 Polymer-containing vaccine formulation (Sample numbers A#03 to A#19)

The antigen stock solution and the polymer-containing composition were mixed in a 1:1 (volume ratio) to obtain a polymer-containing vaccine formulation with an antigen concentration of 0.0033 w/v%.

### (3)-3 Publicly known vaccine adjuvant-containing vaccine formulation (Sample numbers A#20 to A#23)

The antigen stock solution and the publicly known vaccine adjuvant-containing composition were mixed in a 1:1 volume ratio to obtain a polymer-containing vaccine formulation with an antigen concentration of 0.0033HA w/v%.

### 2.Antibody Production Induction Test in Mice (Intranasal mucosal administration)

### (Method)

Each vaccine formulation (A#01, A#03 to A#23) was administered once to both nostrils of BALB/c mice (female, 6 weeks old) at 15 µL per nostril (a total of 30 µL: antigen amount 1 µgHA) (3 mice per group). Three weeks after administration, bronchoalveolar lavage fluid was collected, and the ability to induce antibody production was analyzed by measuring the antibody titer of influenza HA antigen-specific IgA in the bronchoalveolar lavage fluid. The dosages of the polymers/ publicly known adjuvants are shown in the table below.

### (Results)

The results (average value of n=3) are shown in the table below.

### (Discussion)

The vaccine formulations A#03 to A#10, which contain a polyacrylic acid homopolymer with a carboxyl group content of 60.5% by mass, showed induction of antibody production.

On the other hand, the polymer-free vaccine formulation (sample number A#01) (which also does not contain a publicly known vaccine adjuvant) and other polymer-containing vaccine formulations (A#11 to A#19) did not show induction of antibody production.

In A#05, A#06, A#09, and A#10, which contain an influenza split antigen and a crosslinked polyacrylic acid which is an acidic polymer, with a carboxyl group content of 60.5% by mass, with a mode diameter of particles of 8.3 to 9.0 µm, not subjected to shear, a high production of influenza HA antigen-specific IgA was observed.

In A#03, A#07, and A#08, which contain a crosslinked polyacrylic acid which is an acidic polymer, with a carboxyl group content of 60.5% by mass, with a mode diameter of particles of 0.64 to 1.3 µm, subjected to shear, a high production of influenza HA antigen-specific IgA was also observed.

| Sample number | Concentration of polymer/publicly known adjuvant relative to the total amount of vaccine formulation (w/v%) | Bronchoalveolar lavage fluid influenza HA antigen-specific IgA (ng/mL) |
|---|---|---|
| | [Dosage (µg/dose)] | |
| A#01 | 0 | 0.0 |
| A#03 | 0.275 % [82.5 µg] | 943.8 |
| A#04 | 0.275 % [82.5 µg] | 108.8 |
| A#05 | 0.275 % [82.5 µg] | 837.5 |
| A#06 | 0.275 % [82.5 µg] | 605.8 |
| A#07 | 0.175 % [52.5 µg] | 363.4 |
| A#08 | 0.175 % [52.5 µg] | 268.3 |
| A#09 | 0.175 % [52.5 µg] | 777.9 |
| A#10 | 0.175 % [52.5 µg] | 360.5 |
| A#15 | 0.50 % [150 µg] | 0.0 |
| A#16 | 0.10 % [30 µg] | 0.0 |
| A#11 | 0.40 % [120.0 µg] | 0.0 |
| A#12 | 0.40 % [120.0 µg] | 0.0 |
| A#13 | 0.125 % [37.5 µg] | 0.0 |
| A#14 | 12.0 % [3600 µg] | 0.0 |
| A#17 | 7.50 % [2250 µg] | 0.0 |
| A#18 | 0.50 % [150 µg] | 0.0 |
| A#19 | 0.50 % [150 µg] | 0.0 |
| A#20 | [10 µg] | 0.5 |
| A#21 | [30 µg] | 0.1 |
| A#22 | [10 µg] | 0.5 |
| A#23 | [100 µg] | 208.1 |

### [Test Example 2]

### Antibody Production Induction Test in Mice (Intranasal mucosal administration, SARS-CoV-2 S1 protein)

### 1.Preparation of vaccine formulation

### (1) Preparation of antigen stock solution

SARS-CoV-2 S1 protein was mixed with physiological saline until homogeneous to obtain an antigen stock solution (antigen concentration 0.02 w/v%).

### (2)-1 Preparation of polymer-containing compositions

Each polymer was placed in purified water or an aqueous sodium chloride solution, for some compositions, the resulting mixture was neutralized with sodium hydroxide or L-arginine. The resulting mixture was mixed until homogeneous to obtain a polymer-containing composition. For a polymer-containing composition "with mechanical shear treatment," the obtained polymer-containing composition was subjected to a mechanical shear force using an intermittently-jet-stream-generating-type high-speed spinning-type emulsifying device to adjust the particle size of the polymer in the polymer-containing composition.

### (2)-2 Measurement of mode diameter of polymer particles

The mode diameter of each polymer before mixing with the antigen was measured using a laser diffraction particle size distribution analyzer.

For the polymer-containing compositions subjected to a mechanical shear force, the polymer-containing compositions to which mechanical shear force had been applied was used as a sample to measure the mode diameter of polymer particles using a laser diffraction particle size distribution analyzer (Shimazu SALD-2300, Shimazu-7000).

The table below shows the details of the polymer used in the preparation of the vaccine formulation and the measurement results of the mode diameter of particles of the polymer; the neutralizing agent used in the preparation of the polymer-containing composition, the sodium chloride concentration relative to the total amount of the polymer-containing composition, and whether or not mechanical shear treatment was applied.

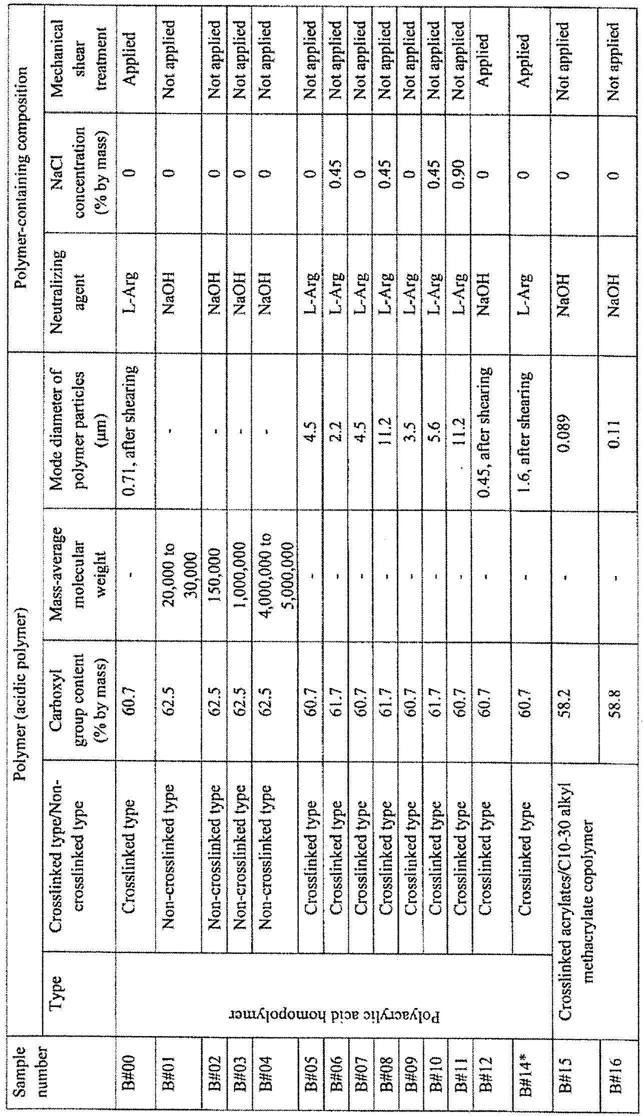

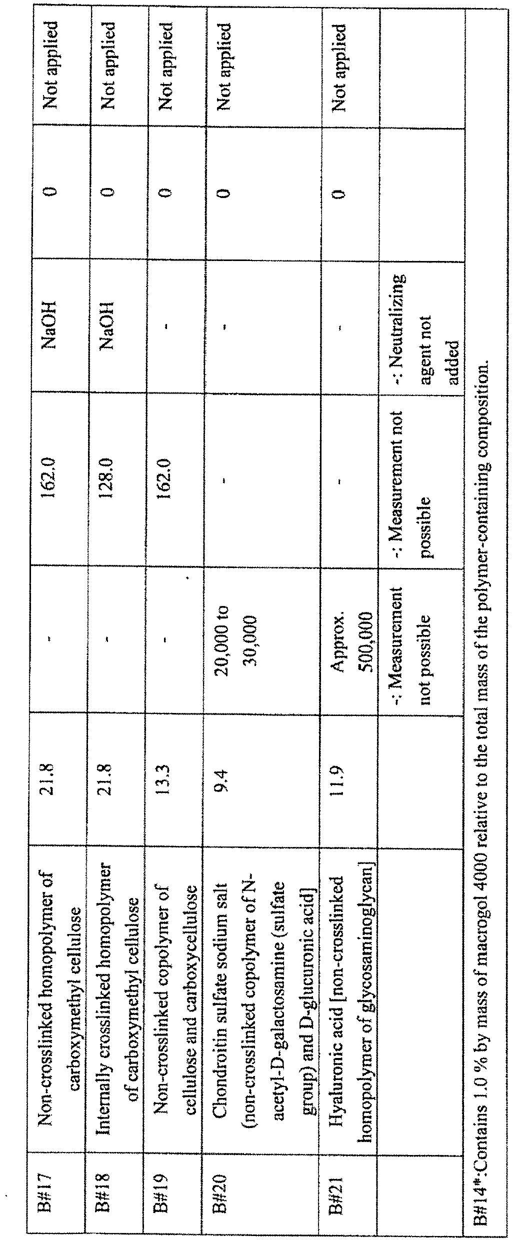

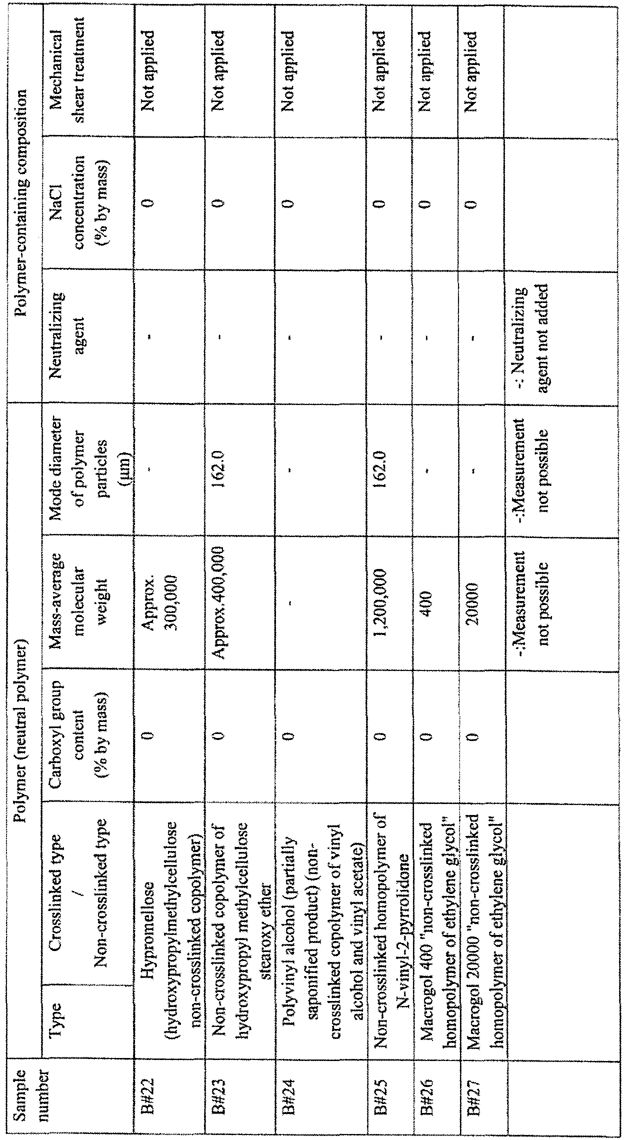

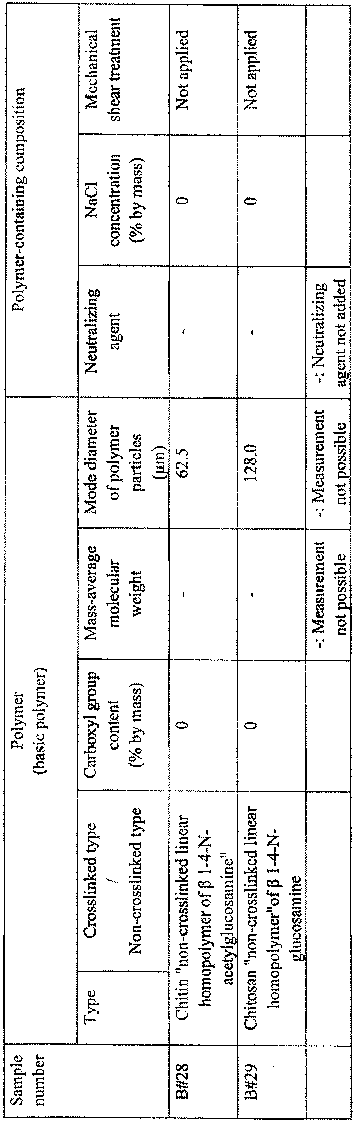

### (3) Preparation of vaccine formulation

The antigen stock solution and the polymer-containing composition were mixed in a 1:1 volume ratio to obtain a vaccine formulation with an antigen concentration of 0.01 w/v%.

### 2. Antibody Production Induction Test in Mice (Intranasal mucosal administration)

### (Method)

The vaccine formulation was administered once to both nostrils of BALB/c mice (female, 6 weeks old) at 15 µL per nostril (total 30 µL: 3 µg of antigen, 105 µg or 165 µg of polymer) (3 mice per group). Three weeks after administration, serum, nasal lavage fluid, and bronchoalveolar lavage fluid were collected and the ability to induce antibody production was analyzed by measuring the antibody titers of SARS-CoV-2 S1 protein-specific IgA in nasal lavage fluid, SARS-CoV-2 S1 protein-specific IgA in bronchoalveolar lavage fluid, and SARS-CoV-2 S1 protein-specific IgG in serum.

As a control, a polymer-free vaccine formulation, with an antigen concentration of 0.01 w/v%, was prepared by mixing the antigen stock solution and physiological saline at a 1:1 volume ratio.

### (Results)

The results (average of n = 10) are shown in the table below.

### (Discussion)

Vaccine formulations B#00 to B#14, which contain a polyacrylic acid homopolymer with a carboxyl group content of 60.7 to 62.5% by mass, showed higher induction of antibody production than the polymer-free vaccine formulation.
B#02, which contains a non-crosslinked with a mass-average molecular weight of 150,000;
B#05 to B#11, which contain a crosslinked polyacrylic acid homopolymer, and have not subjected to a mechanical shear treatment, and have a mode diameter of particles of the polymer of 2.2 to 11.2 µm; and
B#00, which has a mode diameter of particles of the polymer of 0.71 µm, and B#12, which has a mode diameter of particles of the polymer of 0.45 µm, both of which are crosslinked type and have subjected to a mechanical shear treatment,
showed a particularly high enhancement in the induction of antibody production compared to administration of the antigen alone.

| Sample numbers | Polymer concentration in vaccine formulation (% by mass) [Polymer dosage (µg/dose)] | SARS-CoV-2 S1 Protein-specific IgA in bronchoalveolar lavage fluid (titer) | SARS-CoV-2 S1 Protein-specific IgA in nasal lavage fluid (titer) | SARS-CoV-2 S1 Protein -specific IgG1 in serum (titer) | SARS-CoV-2 S1 Protein -specific IgG2a in serum (titer) |
|---|---|---|---|---|---|
| Untreated | | 0 | 0 | 0 | 0 |
| Polymer-free vaccine formulation | 0% [0µg] | 0 | 0 | 1500 | 0 |
| B#00 | 0.55% [165µg] | 667 | 5 | 86667 | 167 |
| B#01 | 0.35% [105µg] | 0 | 0 | 5333 | 0 |
| B#02 | 0.35% [105µg] | 10293 | 117 | 725333 | 53500 |
| B#03 | 0.35% [105µg] | 67 | 1 | 42667 | 333 |
| B#04 | 0.35% [105µg] | 33 | 1 | 34667 | 667 |
| B#05 | 0.35% [105µg] | 40 | 1 | 364000 | 167 |
| B#06 | 0.35% [105µg] | 117 | 2 | 22667 | 2667 |
| B#07 | 0.35% [105µg] | 10 | 1 | 32667 | 167 |
| B#08 | 0.35% [105µg] | 373 | 13 | 22400 | 2667 |
| B#09 | 0.35% [105µg] | 7 | 3 | 10667 | 167 |
| B#10 | 0.35% [105µg] | 133 | 3 | 11333 | 333 |
| B#11 | 0.55% [165µg] | 173 | 12 | 37333 | 333 |
| B#12 | 0.55% [165µg] | 1173 | 37 | 405333 | 6000 |
| B#14* | 0.55% [165µg] | 8960 | 59 | 704000 | 44333 |
| | | | | | |
| B#15 | 0.35% [105µg] | 0 | 0 | 333 | 0 |
| B#16 | 0.35% [105µg] | 60 | 0 | 3000 | 1333 |
| | | | | | |
| B#17 | 0.35% [105µg] | ' 33 | 0 | 3000 | 1000 |
| B#18 | 0.35% [105µg] | 23 | 1 | 4333 | 667 |
| B#19 | 0.35% [105µg] | 0 | 0 | 3667 | 0 |
| B#20 | 0.35% [105µg] | 0 | 1 | 4000 | 0 |
| B#21 | 0.35% [105µg] | 7 | 0 | 1667 | 0 |
| B#22 | 0.35% [105µg] | 0 | 1 | 0 | 0 |
| B#23 | 0.35% [105µg] | 0 | 0 | 333 | 167 |
| B#24 | 0.35% [105µg] | 0 | 0 | 0 | 0 |
| B#25 | 0.35% [105µg] | 0 | 0 | 167 | 0 |
| B#26 | 0.35% [105µg] | 3 | 0 | 0 | 0 |
| B#27 | 0.35% [105µg] | 0 | 0 | 0 | 0 |
| B#28 | 0.35% [105µg] | 0 | 0 | 2667 | 0 |
| B#29 | 0.35% [105µg] | 7 | 0 | 12667 | 0 |

### (Discussion-2)

The ratio of the antibody titer of the samples containing polyacrylic acid homopolymer to the antibody titer of B#00 was calculated. As shown in the table below, it was suggested that B#00, B#02, B#12, and B#14 enhance the production of both IgG and IgA at different sites.

The result of B#14 suggests that the combined use of polyacrylic acid homopolymer and polyethylene glycol synergistically enhances antibody production.

| Sample numbers | Polymer dosage | SARS-CoV-2 S1 Protein -specific IgA in bronchoalveolar lavage fluid | SARS-CoV-2 S1 Protein -specific IgA in nasal lavage fluid | SARS-CoV-2 S1 Protein -specific IgG1 in serum | SARS-CoV-2 S1 Protein -specific IgG2a in serum |
|---|---|---|---|---|---|
| Untreated | | 0.00 | 0.00 | 0.00 | 0.00 |
| Polymer-free vaccine formulation | 0 µg | 0.00 | 0.00 | 0.02 | 0.00 |
| B#00 | 165 µg | 1.00 | 1.00 | 1.00 | 1.00 |
| B#01 | 105 µg | 0.00 | 0.00 | 0.06 | 0.00 |
| B#02 | 105 µg | 15.43 | 23.40 | 8.37 | 320.36 |
| B#03 | 105 µg | 0.10 | 0.20 | 0.49 | 1.99 |
| B#04 | 105 µg | 0.05 | 0.20 | 0.40 | 3.99 |
| B#05 | 105 µg | 0.06 | 0.20 | 4.20 | 1.00 |
| B#06 | 105 µg | 0.18 | 0.40 | 0.26 | 15.97 |
| B#07 | 105 µg | 0.01 | 0.20 | 0.38 | 1.00 |
| B#08 | 105 µg | 0.56 | 2.60 | 0.26 | 15.97 |
| B#09 | 105 µg | 0.01 | 0.60 | 0.12 | 1.00 |
| B#10 | 105 µg | 0.20 | 0.60 | 0.13 | 1.99 |
| B#11 | 165 µg | 0.26 | 2.40 | 0.43 | 1.99 |
| B#12 | 165 µg | 1.76 | 7.40 | 4.68 | 35.93 |
| B#14* | 165 µg | 13.43 | 11.80 | 8.12 | 265.47 |

The ingredients and amounts thereof in the representative examples of the polymer-containing composition prepared in (2)-1 above are shown in the table below.

| Polymer-containing composition used for B#00 | |
|---|---|
| Ingredient | Amount [% by mass] |
| Crosslinked polyacrylic acid homopolymer | 1.10 |
| L-arginine | 2.30 |
| Purified water | 96.60 |
| Total | 100 |

| Polymer-containing composition used for B#02 | |
|---|---|
| Ingredient | Amount [% by mass] |
| Non-crosslinked polyacrylic acid homopolymer | 0.70 |
| Sodium hydroxide | 0.345 |
| Purified water | 98.955 |
| Total | 100 |

| Polymer-containing composition used for B#08 | |
|---|---|
| Ingredient | Amount [% by mass] |
| Crosslinked polyacrylic acid homopolymer | 0.70 |
| L-arginine | 1.52 |
| Sodium chloride | 0.45 |
| Purified water | 97.33 |
| Total | 100 |

| Polymer-containing composition used for B#11 | |
|---|---|
| Ingredient | Amount [% by mass] |
| Crosslinked polyacrylic acid homopolymer | 1.10 |
| L-arginine | 2.30 |
| Sodium chloride | 0.90 |
| Purified water | 95.70 |
| Total | 100 |

| Polymer-containing composition used for B#12 | |
|---|---|
| Ingredient | Amount [% by mass] |
| Crosslinked polyacrylic acid homopolymer | 1.10 |
| Sodium hydroxide | 0.544 |
| Purified water | 98.356 |
| Total | 100 |

The pH and viscosity of representative examples of the vaccine formulations prepared in (3) above are shown in the table below.

| | B#00 | B#02 | B#08 | B#11 | B#12 |
|---|---|---|---|---|---|
| pH | 6.88 | 7.05 | 7.29 | 6.73 | 7.15 |
| Viscosity [mPa·S] | 104 | 10 | 162 | 407 | 131 |

### <Method of measuring viscosity>

Viscosity was measured according to Viscosity measurement by rotational viscometer (Cone-flat plate-type rotational viscometer) as described in Viscosity Determination of the general test method of the Japanese Pharmacopoeia.

### [Test Example 3]

### Antibody Production Induction Test in Mice (Intranasal mucosal administration, ovalbumin (OVA))

### 1.Preparation of vaccine formulation

### (1) Preparation of antigen stock solution

Ovalbumin (OVA, Sigma Grade V) was mixed with phosphate-buffered saline (PBS) until homogeneous to obtain an antigen stock solution (antigen concentration 0.2 w/v%).

### (2)-1 Preparation of polymer-containing compositions used in sample numbers C#01 to C#05

Each polymer was placed in PBS (phosphate buffered saline), neutralized with sodium hydroxide or L-arginine, and the resulting mixture was mixed until homogeneous to obtain a composition.

For polymer-containing compositions "with mechanical shear treatment," the obtained composition was subjected to a mechanical shear force using an intermittently-jet-stream-generating-type high-speed spinning-type emulsifying device to adjust the particle size of the polymer in the polymer-containing composition.

### (2)-2 Preparation of polymer-containing compositions used in sample numbers C#06 to C#22

Each polymer was mixed with PBS (phosphate buffered saline) until homogeneous to obtain a composition.

### (2)-3 Measurement of mode diameter of polymer particles

The mode diameter of each polymer before mixing with the antigen was measured using a laser diffraction particle size distribution analyzer (Shimazu SALD-2300, Shimazu SALD-7000).

For the polymer-containing compositions subjected to a mechanical shear force, the polymer-containing compositions after the mechanical shear force was used as a sample to measure the mode diameter of particles of the polymer with a laser diffraction particle size distribution analyzer.

The table below shows details of the polymer used in the preparation of the vaccine formulation and the measurement results of the mode diameter of particles of the polymer; as well as the neutralizing agent used in the preparation of the polymer-containing composition, the sodium chloride concentration relative to the total amount of the polymer-containing composition, and whether or not mechanical shear treatment was performed.

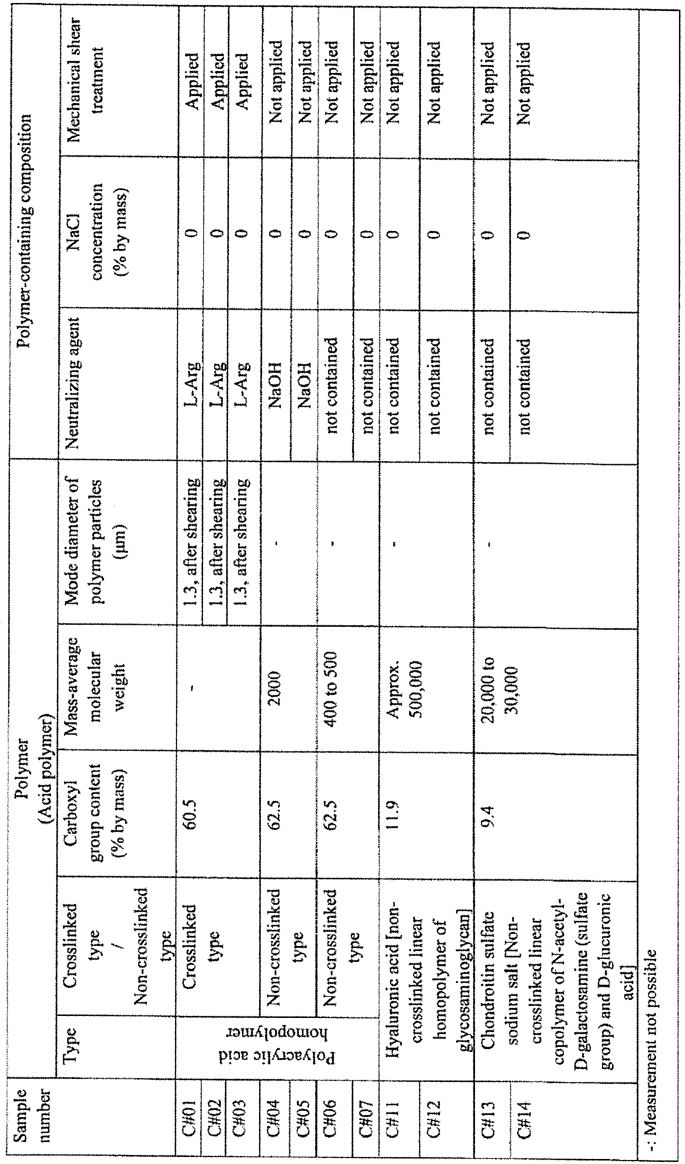

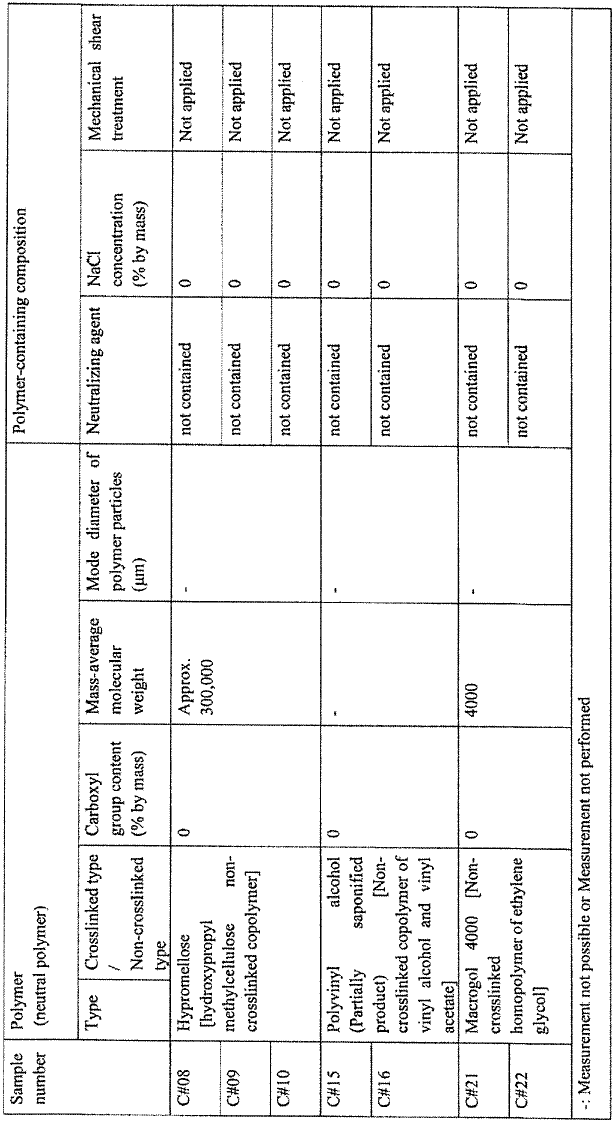

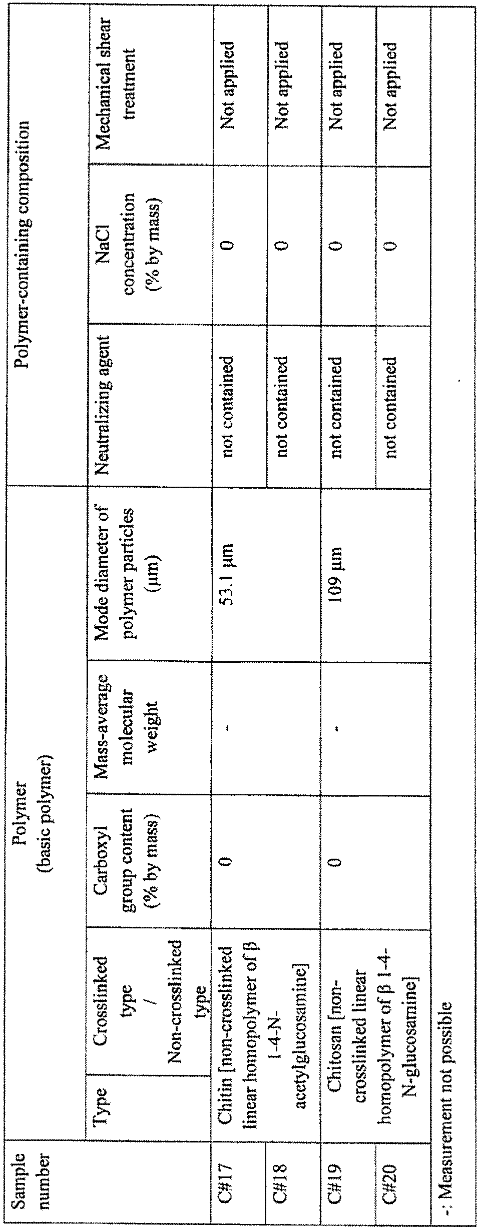

### (3)-1 Polymer-free vaccine formulation (sample number C#00)

The antigen stock solution and PBS (phosphate buffered saline) were mixed in a 1:1 (volume ratio) to obtain a polymer-free vaccine formulation with an antigen concentration of 0.1 w/v%.

### (3)-2 Preparation of polymer-containing vaccine formulation

The antigen stock solution and the polymer-containing composition were mixed in a 1:1 (volume ratio) to obtain a vaccine formulation with an antigen concentration of 0.1 w/v%.

### 2.Antibody Production Induction Test in Mice (Intranasal mucosal administration)

### (Method)

Each vaccine formulation was administered twice at a 2-week interval to BALB/c mice (female, 7 weeks old) at 5 µL each into both nostrils (total 10 µL: antigen amount 10 µg) (10 mice per group). Two weeks after the second administration, blood and nasal lavage fluid were collected, and absorbance was measured using a microplate reader (450 nm) to determine the amounts of OVA-specific IgA in the nasal lavage fluid and OVA-specific IgG in the blood.

### (Results)

The results (average of n = 10) are shown in the table below.

### (Discussion)

C#01 to C#07, which contain a cross-linked polyacrylic acid homopolymer (carboxyl group content of 60.5-62.5% by mass), showed an improved production of OVA-specific IgG in blood compared to C#00, which does not contain any polymer.

C#01 to C#03, which contain a cross-linked polyacrylic acid homopolymer with a carboxyl group content of 60.5% by mass, showed improved antibody production in nasal lavage fluid OVA-specific IgA and blood OVA-specific IgG, compared to C#00, which does not contain any polymer.

Compared to C#00, which does not contain any polymer, a strong antibody production enhancing effect was observed in C#01, C#02, and C#03, which contain an ovalbumin antigen and a cross-linked acrylic acid-based homopolymer with a carboxyl group content of 60.5% by mass, has been subjected to mechanical shear, and has a polymer particle diameter of 1.3 µm.

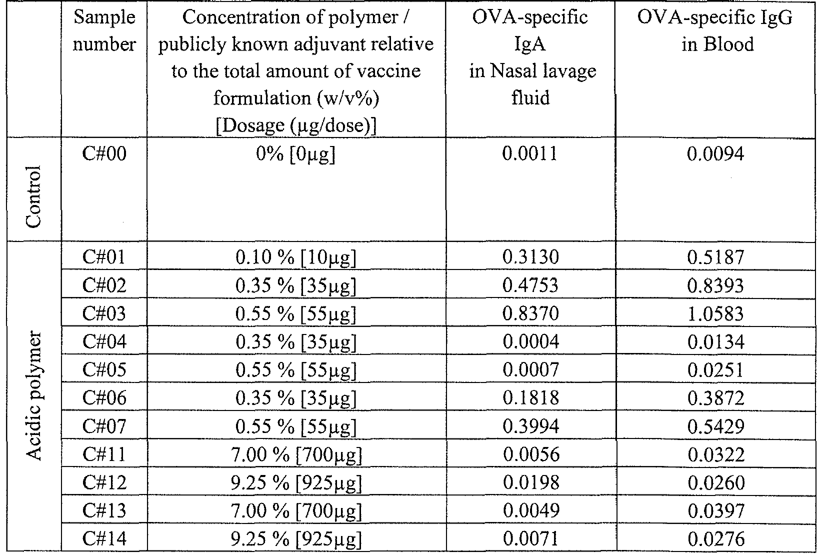

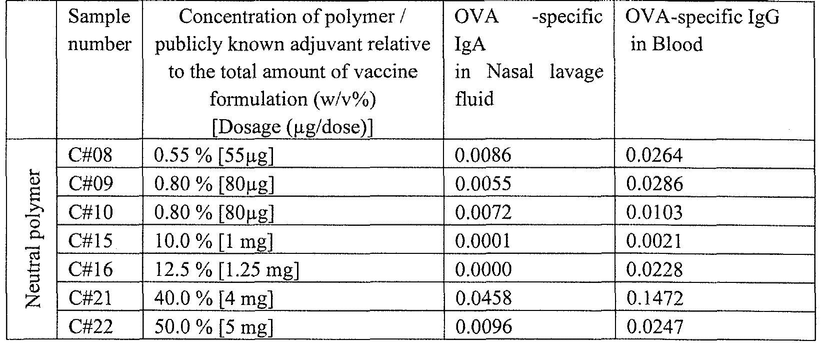

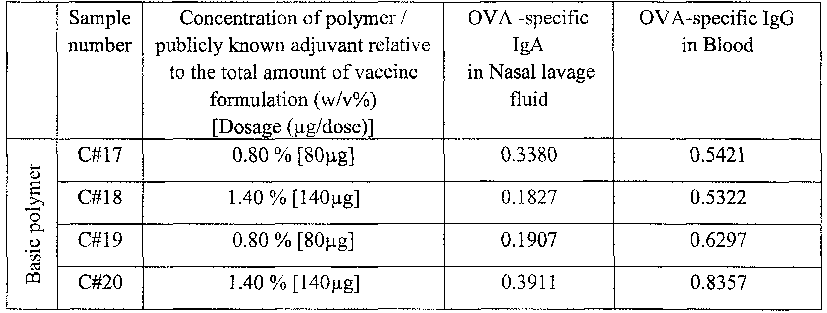

### [Test Example 4] Antibody Production Induction Test in Mice (subcutaneous administration, SARS-CoV-2 S1 protein)

### 1.Preparation of vaccine formulation

### (1) Polymer-free vaccine formulation (Sample number IA#00)

SARS-CoV-2 S1 protein and physiological saline (NaCl concentration 0.9 w/v%) were mixed until homogeneous to obtain a vaccine formulation. Each 200 µL vaccine formulation contained 3 µg of SARS-CoV-2 S1 protein.

### (2) Polymer-containing vaccine formulation (sample number IA#01)

SARS-CoV-2 S1 protein and a crosslinked polyacrylic acid homopolymer (carboxyl group content 61.5 % by mass, mode diameter of polymer particles 2.2 µm) were added to physiological saline (NaCl concentration 0.9 w/v%), neutralized with sodium hydroxide, and mixed until homogeneous to obtain a vaccine formulation. Each 200 µL vaccine formulation contained 3 µg of SARS-CoV-2 S1 protein and 600 µg of the cross-linked polyacrylic acid homopolymer.

### 2.Antibody Production Induction Test in Mice (subcutaneous administration)

Each vaccine formulation (200 µL) was subcutaneously administered twice at two-week intervals into BALB/c mice (female, 6 weeks old) (4 mice per group). Serum was collected two weeks after the final administration, and the antibody production induction ability was analyzed by measuring the antibody titer of SARS-CoV-2 S1 protein-specific IgG in the serum.

### (Results)

The results (average value of n=4) are shown in the table below.

| Sample number | SARS-CoV-2 S1 Protein dosage (µg/dose) | Concentration of polymer relative to the total amount of vaccine formulation [Dosage (µg/dose)] | SARS-CoV-2 S1 Protein -specific IgG1 in serum (titer) | SARS-CoV-2 S1 Protein -specific IgG2a in serum (titer) |
|---|---|---|---|---|
| IA#00 (Control) | 3 µg | 0 w/v% [0 µg] | 22000 | 625 |
| IA#01 | 3 µg | 0.3 w/v% [600 µg]] | 13312000 | 208000 |

### (Discussion)

When the cross-linked polyacrylic acid homopolymer (carboxyl group content: 61.5% by mass) was administered subcutaneously together with the antigen SARS-CoV-2 S1 protein, it improved the antibody production-inducing ability of the antigen.

### [Test Example 5] Antibody Production Induction Test in Mice (subcutaneous administration, Influenza split antigen)

### 1.Preparation of vaccine formulation

### (1) Polymer-free vaccine formulation (Sample numbers IB#00)

Influenza split antigen [H1N1] and physiological saline (NaCl concentration 0.9 w/v%) were mixed until homogeneous to obtain a vaccine formulation.

Each 200 µL vaccine formulation contained 1 µg HA of influenza split antigen.

### (2) Polymer-containing vaccine formulation (sample numbers IB#01, IB#02)

A cross-linked polyacrylic acid homopolymer (carboxyl group content 60.7% by mass) was added to physiological saline (NaCl concentration 0.9% w/v), neutralized with sodium hydroxide, and the resulting mixture was mixed until homogeneous. The resulting mixture was subjected to a mechanical shear force using an intermittently-jet-stream-generating-type high-speed spinning-type emulsifying device to adjust the particle size of the polymer in the polymer-containing composition. The mode diameter of particles of the polymer contained in the polymer-containing composition after the mechanical shearing treatment was 0.71 µm. The obtained mechanically sheared polymer-containing composition and the influenza split antigen were mixed until homogeneous to obtain a vaccine formulation.

Each 200 µL vaccine formulation of sample number IB#01 contained 1 µgHA of influenza split antigen and 60 µg of the cross-linked polyacrylic acid homopolymer.

Each 200 µL vaccine formulation of sample number IB#02 contained 1 µgHA of influenza split antigen and 600 µg of the cross-linked polyacrylic acid homopolymer.

### 2.Antibody Production Induction Test in Mice (subcutaneous administration)

The vaccine formulation (200 µL) was subcutaneously administered twice at a 2-week interval into BALB/c mice (female, 6 weeks old) (4 mice per group). Serum was collected 2 weeks after the final administration and the amount of influenza split antigen-specific antibody in the serum was measured to analyze the antibody production induction ability.

### (Results)

The results (average value of n=4) are shown in the table below.

### (Discussion)

When the highly crosslinked polyacrylic acid homopolymer (carboxyl group content: 60.7% by mass, polymer particle mode diameter adjusted to 0.71 µm by shearing treatment) was administered subcutaneously together with an influenza split vaccine antigen, it improved the antibody production-inducing ability of the antigen.

| Sample numbers | Influenza split antigen dosage (µgHA/ dose) | Concentration of polymer relative to the total amount of vaccine formulation [Dosage (µg/dose)] | Influenza split antigen -specific IgG1 in serum (ng/mL) | Influenza split antigen -specific IgG2a in serum (ng/mL) |
|---|---|---|---|---|
| IB#00 (Control) | 1 µgHA | 0 w/v% [0µg] | 45143 | 33985 |
| IB#01 | 1 µgHA | 0.03 w/v% [60µg] | 207113 | 216734 |
| IB#02 | 1 µgHA | 0.3 w/v% [600µg] | 209326 | 1224627 |

### [Test Example 6] Antibody Production Induction Test in Mice (Intranasal mucosal administration, Influenza split antigen, part 2)

### 1.Preparation of vaccine formulation

### (1) Preparation of antigen stock solution

Influenza split antigen [H1N1] was mixed with physiological saline until homogeneous to obtain an antigen stock solution (antigen concentration 0.02HA w/v%).

### (2)-1 Preparation of polymer-containing compositions used in sample numbers D#01 to D#08

Each polymer was placed in purified water, and if pH adjustment was required, it was neutralized with sodium hydroxide or L-arginine, and mixed until homogeneous to obtain a composition.

For polymer-containing compositions "with mechanical shear treatment", the obtained composition was subjected to mechanical shear force using an intermittently-jet-stream-generating-type high-speed spinning-type emulsifying device, and the particle size (mode diameter) of the polymer in the composition was adjusted to about 0.7 to 0.9 µm.

Next, for the polymer-containing compositions containing other additives, the other additives (polysorbate 80; glycerin; macrogol 4000; macrogol 4000 and polysorbate 80) were added.

### (2)-2 Measurement of mode diameter of polymer particles

The mode diameter of each polymer before mixing with the antigen (and the other additives) was measured using a laser diffraction particle size distribution analyzer (Shimazu SALD-7000), a dynamic light scattering particle size distribution analyzer (UPT-UT 151). For the non-crosslinked polyacrylic acid homopolymers, since it was impossible to measure them with a laser diffraction particle size distribution analyzer, it was measured with a dynamic light scattering particle size distribution analyzer (UPT-UT 151).

For the polymer-containing compositions to which mechanical shear force had been applied, the polymer-containing compositions after mechanical shear force treatment (for the polymer-containing compositions containing the other additives, before the addition of the other additives) were used as samples to measure the mode diameter of polymer particles using a laser diffraction particle size distribution analyzer.

The table below shows details of the polymer used in the preparation of the vaccine formulation and the measurement results of the mode diameter of particles of the polymer; as well as the neutralizing agent used in the preparation of the polymer-containing composition which was prepared using the polymer, the sodium chloride concentration relative to the total amount of the polymer-containing composition, the types of other additives and the concentrations thereof relative to the total amount of the polymer-containing composition, and whether or not mechanical shear treatment was performed.

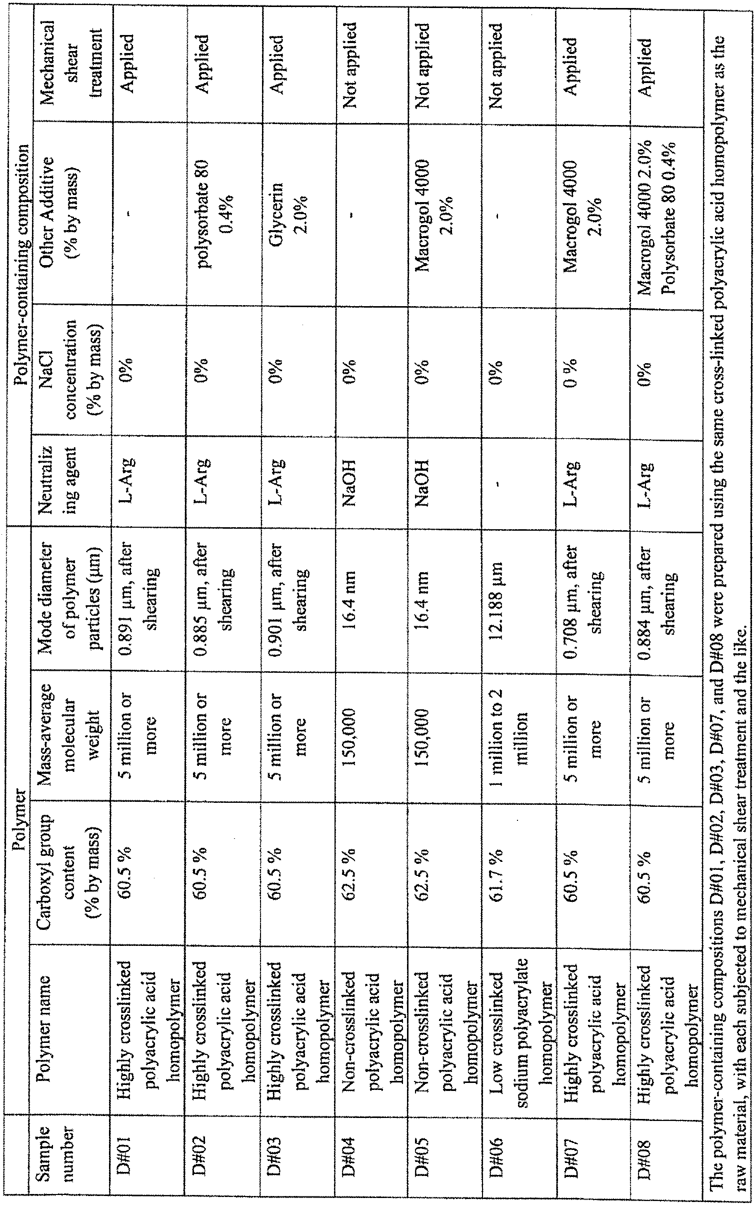

### (3)-1 Polymer-containing vaccine formulation (sample numbers D#01 to D#08)

The antigen stock solution and the polymer-containing composition were mixed at a 1:1 (volume ratio) to obtain a polymer-containing vaccine formulation with an antigen concentration of 0.01 HA w/v%.

### 2.Antibody Production Induction Test in Mice (Intranasal mucosal administration)

### (Method)

Each vaccine formulation (D#01 to D#08) was administered to BALB/c mice (female, 6 weeks old) once via both nostrils, 5 µL each (total 10 µL: antigen amount 1 µg) (4 mice per group). Two weeks after administration, nasal lavage fluid and bronchoalveolar lavage fluid were collected. The antibody production induction ability was analyzed by measuring the antibody titers of influenza HA antigen-specific IgA in the nasal lavage fluid and bronchoalveolar lavage fluid. The dosage of the polymer is shown in the table below.

### (Results)

The results (average value of n=4 are shown in the table below.

| Sample numbers | Concentration of polymer adjuvant relative to the total amount of vaccine formulation (w/v%) [Dosage (µg/dose)] | Influenza HA antigen -specific IgA in nasal lavage fluid (OD450 value) | Influenza HA antigen -specific IgA in bronchoalveolar lavage fluid (OD450 value) |
|---|---|---|---|
| D#01 | 0.55 % [55µg] | 0.7534 | 0.4279 |
| D#02 | 0.55 % [55µg] | 3.3563 | 1.1104 |
| D#03 | 0.55 % [55µg] | 2.2735 | 0.5739 |
| D#04 | 0.55 % [55µg] | 0.2194 | 0.0662 |
| D#05 | 0.55 % [55µg] | 0.2583 | 0.0831 |
| D#06 | 0.55 % [55µg] | 0.3427 | 0.0854 |
| D#07 | 0.55 % [55µg] | 1.8014 | 0.5957 |
| D#08 | 0.55 % [55µg] | 1.7496 | 0.3251 |
| Untreated | - | 0.0648 | 0.0716 |

### (Discussion)

By administering 5µL per nostril (10µL in total) and confining the immunization to the nasal cavity, the differences in the ability of each polymer to induce antibody production were more clearly evaluated.

Compared with the untreated control group, increased antibody production induction ability was confirmed in the non-crosslinked polyacrylic acid homopolymer groups (D#04, D#05) and the low crosslinked polyacrylic acid homopolymer group (carboxyl group content 61.7%) having a molecular weight of 1 to 2 million (D#06).

D#01, which contains a highly crosslinked polyacrylic acid homopolymer (carboxyl group content 60.5%) having a molecular weight of 5 million or more, has been subjected to mechanical shear, showed a significant increase in the ability to induce antibody production.

The highly crosslinked polyacrylic acid homopolymer (carboxyl group content 60.5%) having a molecular weight of 5 million or more, subjected to mechanical shear, when it was combined with glycerin or macrogol 4000, which are polyhydric alcohols (D#03, D#07, D#08), polysorbate 80, which is a nonionic surfactant (D#02, D#08), showed further increase in the ability to induce antibody production.

In particular, the highly crosslinked polyacrylic acid homopolymer having a molecular weight of 5 million or more, subjected to mechanical shear, when it was combined with a nonionic surfactant, polysorbate 80 (D#02) showed the production of influenza HA antigen-specific IgA in both nasal lavage fluid and bronchoalveolar lavage fluid, which showed that the ability to induce antibody production was dramatically increased.

### [Test Example 7] Antibody Production Induction Test in Mice (Intranasal mucosal administration, SARS-CoV-2 S1 protein, part 2)

### 1.Preparation of vaccine formulation

### (1) Preparation of antigen stock solution

SARS-CoV-2 S1 protein was mixed with physiological saline until homogeneous to obtain an antigen stock solution (antigen concentration 0.02 w/v%).

### (2)-1 Preparation of polymer-containing compositions used in sample numbers E#01 to E#13

Each polymer was placed in purified water or an aqueous sodium chloride solution, neutralized with sodium hydroxide or L-arginine, and mixed until homogeneous to obtain a composition. For polymer-containing compositions "with mechanical shear treatment", the obtained composition was subjected to a mechanical shear force using an intermittently-jet-stream-generating-type high-speed spinning-type emulsifying device, and the particle size (mode diameter) of the polymer in the polymer-containing composition was adjusted to about 0.7 to 0.9 µm. Next, for the polymer-containing compositions containing other additive(s), the other additive(s) were added thereto.

### (2)-2 Measurement of mode diameter of polymer particles

The mode diameter of each polymer before mixing with the antigen (and other additives) was measured using a laser diffraction particle size distribution analyzer (Shimazu SALD-2300, Shimazu SALD-7000).

For the polymer-containing compositions to which mechanical shear force had been applied, the polymer-containing compositions after the application of mechanical shear force (for the polymer-containing compositions containing other additives, before the addition of the other additives) were used as samples to measure the mode diameter of particles of the polymer using a laser diffraction particle size distribution analyzer.

The table below shows details of the polymer used in the preparation of the vaccine formulation and the measurement results of the mode diameter of particles of the polymer; as well as the neutralizing agent used in the preparation of the polymer-containing composition which was prepared using the polymer, the sodium chloride concentration relative to the total amount of the polymer-containing composition, the types of other additives and concentrations thereof relative to the total amount of the polymer-containing composition, and whether or not mechanical shear treatment was performed.

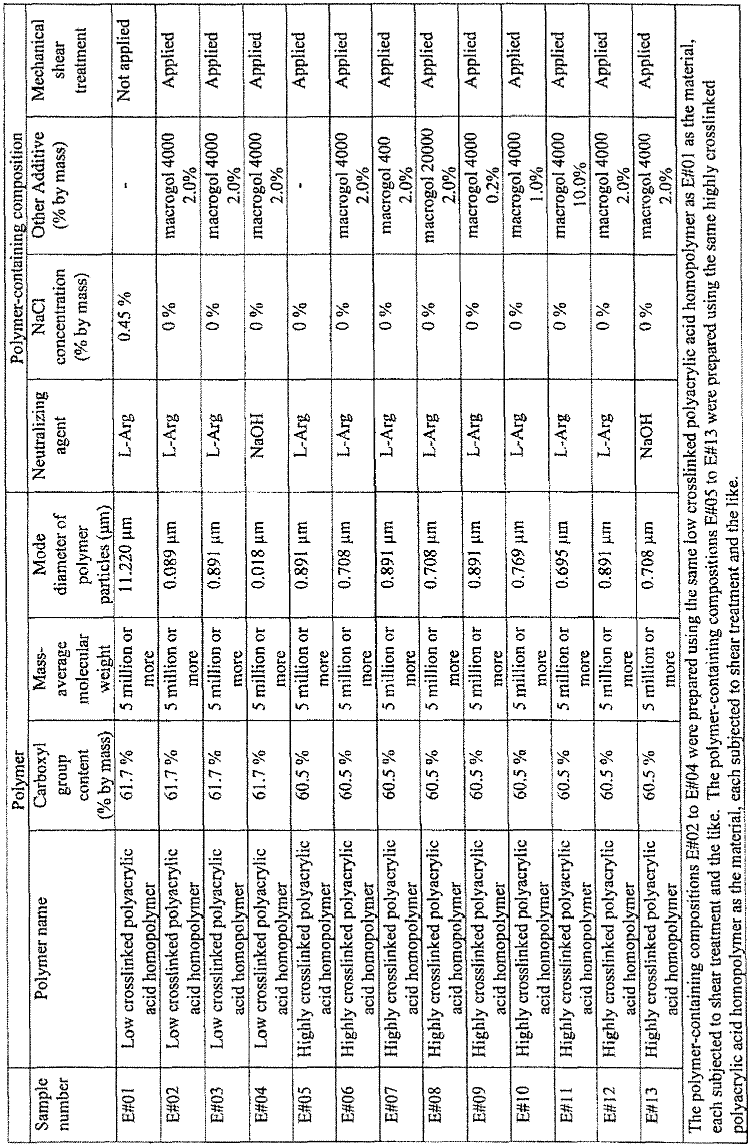

### (3)-1 Polymer-containing vaccine formulation (sample numbers E#01 to E#13)

The antigen stock solution and the polymer-containing composition were mixed in a 1:1 (volume ratio) to obtain a polymer-containing vaccine formulation with an antigen concentration of 0.01 HA w/v%.

### 2.Antibody Production Induction Test in Mice (Intranasal mucosal administration)

### (Method)

Each vaccine formulation (E#01 to E#13) was administered once to both nostrils of BALB/c mice (female, 6 weeks old) at 15 µL per nostril (total 30 µL: antigen amount 3 µg) (4 mice per group). Three weeks after administration, nasal lavage fluid and bronchoalveolar lavage fluid were collected and the antibody titers of SARS-CoV-2 S1 protein-specific IgA in the nasal lavage fluid and SARS-CoV-2 S1 protein-specific IgA in the bronchoalveolar lavage fluid were measured to analyze the antibody production induction ability. The dosage of the polymer is shown in the table below.

### (Results)

The results (average value of n=4) are shown in the table below.

| Sample numbers | Concentration of polymer adjuvant relative to the total amount of vaccine formulation (w/v%) [Dosage (µg/dose)] | SARS-CoV-2 S1 protein antigen -specific IgA in nasal lavage fluid (titer) | SARS-CoV-2 S1 protein antigen -specific IgA in bronchoalveolar lavage fluid (titer) |
|---|---|---|---|
| E#01 | 0.55 % [165µg] | 15 | 570 |
| E#02 | 0.55 % [165µg] | 45 | 9280 |
| E#03 | 0.55 % [165µg] | 45 | 4640 |
| E#04 | 0.55 % [165µg] | 35 | 9120 |
| E#05 | 0.55 % [165µg] | 45 | 685 |
| E#06 | 0.55 % [165µg] | 85 | 9315 |
| E#07 | 0.55 % [165µg] | 70 | 2060 |
| E#08 | 0.55 % [165µg] | 75 | 17920 |
| E#09 | 0.55 % [165µg] | 85 | 4480 |
| E#10 | 0.55 % [165µg] | 50 | 9120 |
| E#11 | 0.55 % [165µg] | 60 | 13760 |
| E#12 | 1.00 % [300µg] | 35 | 3440 |
| E#13 | 0.55 % [165µg] | 25 | 7040 |

### (Discussion)

Whether a low crosslinked polyacrylic acid homopolymer or a highly crosslinked polyacrylic acid homopolymer, the addition of polyethylene glycol resulted in a significant increase in the ability to induce the production of SARS-CoV-2-specific IgA antibodies, especially in bronchoalveolar lavage fluid, compared to formulations without polyethylene glycol (E#01, E#05).

In the case of a low crosslinked polyacrylic acid homopolymer, while the effect of adding polyethylene glycol was observed, controlling the particle size of the polymer particles to 0.1 µm or less resulted in an increased the ability to induce antibody production.

In the case of a highly crosslinked polyacrylic acid homopolymer, the effect of adding polyethylene glycol increased the antibody production-inducing ability, regardless of the polymer particle size as far as it was adjusted to 0.7 to 0.9 µm of polymer particles (comparison between E#05 and E#06).

In the case of a highly crosslinked polyacrylic acid homopolymer, when the concentration of polyethylene glycol was the same (1.0%), the ability of inducing the production of SARS-CoV-2-specific IgA antibody in bronchoalveolar lavage fluid increased with increasing molecular weight of polyethylene glycol (macrogol 400 [E#07]<macrogol 4000 [E#06]<macrogol 20000 [E#08]).

In the case of a highly crosslinked polyacrylic acid homopolymer, when polyethylene glycol 4000 was used, the ability to induce the production of SARS-CoV-2-specific IgA antibody in the bronchoalveolar lavage fluid increased as the added amount increased from 0.1% [E#09], 0.5% [E#10], 1.0% [E#06], and 5.0% [E#11].

In the case of a highly crosslinked polyacrylic acid homopolymer, no increase in antibody production induction ability was observed even when the amount of polymer was increased [E#12] or when the neutralizing agent was changed [E#13].

### INDUSTRIAL APPLICABILITY

The present invention polymer may be used as a vaccine adjuvant.

## Claims

1. A vaccine adjuvant agent comprising a polymer in which a constitutional unit is derived from acrylic acid, wherein the polymer has a carboxyl group content of 60.0 to 62.5 % by mass.

2. The vaccine adjuvant agent according to Claim 1, wherein the polymer is polyacrylic acid and/or a salt thereof.

3. The vaccine adjuvant agent according to Claim 1 or 2, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is 0.05 to 10 µm.

4. The vaccine adjuvant agent according to any one of Claims 1 to 3, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is adjusted to 0.05 to 10 µm by applying a mechanical shear force.

5. The vaccine adjuvant agent according to Claim 4, wherein the carboxyl group content is 60.0 to 61.0 % by mass as calculated for the free form of the polyacrylic acid.

6. The vaccine adjuvant agent according to Claim 5, wherein a number-based mode diameter of particles of the polymer is adjusted to 0.1 to 2.0 µm by applying a mechanical shear force.

7. The vaccine adjuvant agent according to Claim 4, wherein the carboxyl group content is more than 61.0 % by mass and less than or equal to 62.0% by mass as calculated for the free form of the polyacrylic acid.

8. The vaccine adjuvant agent according to Claim 7, wherein a number-based mode diameter of particles of the polymer is adjusted to 0.05 to 0.1 µm by applying a mechanical shear force.

9. The vaccine adjuvant agent according to Claim 1 or 2, wherein the polymer is a crosslinked polyacrylic acid and/or a salt thereof; the carboxyl group content is 60.0 to 62.0 % by mass as calculated for the free form of the polyacrylic acid; and a number-based mode diameter of particles of the polymer is 0.1 to 50 µm.

10. The vaccine adjuvant agent according to Claim 9, wherein the polymer has not been subjected to a mechanical shear force.

11. The vaccine adjuvant agent according to Claim 1 or 2, wherein the polymer is a non-crosslinked polyacrylic acid and/or a salt thereof, and, the polymer has a mass-average molecular weight ranging from 50,000 to 300,000.

12. The vaccine adjuvant agent according to Claim 11, wherein the carboxyl group content is 62.0 to 62.5 % by mass as calculated for the free form of the polyacrylic acid.

13. The vaccine adjuvant agent according to any one of Claims 1 to 12, further comprising water.

14. The vaccine adjuvant agent according to any one of Claims 3 to 9 and 13, wherein
the vaccine adjuvant agent comprises the polymer and water, and optionally comprises at least one additive selected from the group consisting of sodium chloride, sodium hydroxide, and L-arginine, and
the number-based mode diameter of particles of the polymer is adjusted by applying a mechanical shear force to a composition consisting of the polymer and the water, provided that, when the vaccine adjuvant agent comprises the at least one additive, the composition may comprise the at least one additive.

15. The vaccine adjuvant agent according to any one of Claims 1 to 14, wherein sodium chloride is comprised in an amount of 0.1 to 1.5 % by mass based on the total mass of the vaccine adjuvant agent.

16. The vaccine adjuvant agent according to any one of Claims 1 to 15, wherein the vaccine adjuvant agent further comprises sodium hydroxide and/or L-arginine

17. The vaccine adjuvant agent according to any one of Claims 1 to 16, wherein the vaccine adjuvant agent further comprises L-arginine

18. The vaccine adjuvant agent according to any one of Claims 1 to 17, wherein the vaccine adjuvant agent has pH of 6 to 8.

19. The vaccine adjuvant agent according to any one of Claims 1 to 18, wherein the polymer is comprised in an amount of 0.01 to 50 % by mass based on the total mass of the vaccine adjuvant agent.

20. The vaccine adjuvant agent according to any one of Claims 1 to 19, wherein the vaccine adjuvant agent further comprises at least one nonionic surfactant selected from the group consisting of an ether-type nonionic surfactant and an ester-ether type nonionic surfactant.

21. The vaccine composition according to Claim 20, wherein the nonionic surfactant is polysorbate 80.

22. The vaccine adjuvant agent according to any one of Claims 1 to 21, wherein the vaccine adjuvant agent further comprises at least one polyhydric alcohol selected from the group consisting of polyethylene glycol, glycerin, propylene glycol, and 1,3-butylene glycol.

23. The vaccine adjuvant agent according to Claim 22, wherein the polyhydric alcohol is polyethylene glycol.

24. The vaccine adjuvant agent according to any one of Claims 1 to 23, for enhancing an immune response to a pathogen-derived antigen.

25. The vaccine adjuvant agent according to Claim 24, wherein a disease caused by the pathogen is a respiratory disease.

26. The vaccine adjuvant agent according to any one of Claims 1 to 25, for enhancing an immune response to an antigen derived from an enveloped virus.

27. The vaccine adjuvant agent according to any one of Claims 1 to 26, for enhancing an immune response to an antigen derived from at least one virus selected from the group consisting of coronavirus and influenza virus.

28. The vaccine adjuvant agent according to any one of Claims 1 to 27, wherein the vaccine adjuvant agent is for administration via an injection route or a mucosal route.

29. The vaccine adjuvant agent according to any one of Claims 1 to 28, wherein the dosage of the polymer per administration is 1 µg to 100 mg.

30. A method for enhancing an immune response to an antigen, comprising administering to a subject in need thereof the vaccine adjuvant agent according to any one of Claims 1 to 29 in an amount effective as a vaccine adjuvant.

31. Use of the vaccine adjuvant agent according to any one of Claims 1 to 29 for enhancing an immune response to an antigen.

32. A method for inducing an immune response to an antigen, comprising administering to a subject in need thereof the vaccine adjuvant agent according to any one of Claims 1 to 29 in an amount effective as a vaccine adjuvant in combination with the antigen.

33. Use of the vaccine adjuvant agent according to any one of Claims 1 to 29 for inducing an immune response to an antigen.

34. A method for preventing a disease caused by a pathogen, comprising administering to a subject in need thereof the vaccine adjuvant agent according to any one of Claims 1 to 29 in an amount effective as a vaccine adjuvant in combination with an antigen derived from the pathogen.

35. Use of the vaccine adjuvant agent according to any one of Claims 1 to 29 for preventing a disease caused by a pathogen.

36. Use of the vaccine adjuvant agent according to any one of Claims 1 to 29 in the manufacture of a medicament for enhancing an immune response to an antigen, inducing an immune response to an antigen, or preventing a disease caused by a pathogen.

37. A vaccine composition comprising (i) the vaccine adjuvant agent according to any one of Claims 1 to 29, and (ii) an antigen.

38. A vaccine composition comprising:
(i) a polymer in an amount effective as a vaccine adjuvant, wherein a constitutional unit of the polymer is derived from acrylic acid, and the polymer has a carboxyl group content of 60.0 to 62.5 % by mass, and
(ii) an antigen.

39. The vaccine composition according to Claim 37 or 38, wherein the antigen is a pathogen-derived antigen.

40. The vaccine composition according to Claim 39, wherein a disease caused by the pathogen is a respiratory disease

41. The vaccine composition according to any one of Claims 37 to 40, wherein the antigen is an antigen derived from an enveloped virus.

42. The vaccine composition according to any one of Claims 37 to 41, wherein the vaccine composition is for administration via an injection route or a mucosal route.

43. The vaccine composition according to any one of Claims 37 to 42, wherein the polymer is comprised in a mass ratio of the antigen: the polymer = 1:0.1 to 1 :1000.

44. A method for inducing an immune response to the antigen, comprising administering to a subject in need thereof an effective amount of the vaccine composition according to any one of Claims 37 to 43.

45. Use of the vaccine composition according to any one of Claims 37 to 43 for inducing an immune response to the antigen.

46. A method for preventing a disease caused by a pathogen, comprising administering to a subject in need thereof an effective amount of the vaccine composition according to any one of Claims 37 to 43.

47. Use of the vaccine composition according to any one of Claims 37 to 43 for preventing a disease caused by a pathogen.
